# EUROPEAN PATENT SPECIFICATION

(11) **EP 2 120 782 B1**
(45) Date of publication and mention of the grant of the patent: **10.10.2018**
(21) Application number: 08712943.3
(22) Date of filing: 03.01.2008
(51) Int. Cl.: A61F 2/00, A61B 17/04

(54) **PELVIC IMPLANT**
PELVIC IMPLANTAT
IMPLANT PELVIEN

(30) Priority: 03.01.2007 US 883253 P; 05.01.2007 US 883709 P
(43) Date of publication of application: 25.11.2009
(73) Proprietor: Boston Scientific Scimed, Inc., Maple Grove, MN 55311 (US)
(72) Inventor: GOZZI, Christian, I-22 Via Fiumes (IT); REHDER, Peter, A-6080 Igls (AT); ROYCHOWDHURY, Suranjan, Plymouth, Minnesota 55441 (US)
(74) Representative: Pfenning, Meinig & Partner mbB
(86) International application number: PCT/US2008/000033
(87) International publication number: WO 2008/085825

(56) References cited:
- WO-A-99/59477
- WO-A-2005/122954
- WO-A-2006/108045
- WO-A-2007/097994

## Description

### Priority Claim

### Field of the Invention

The invention relates to a pelvic implant assembly.

### Background

Men, women, and children of all ages can suffer from urinary and fecal incontinence or involuntary loss of urinary and bowel control. Their lives are perpetually interrupted by thoughts of ensuring that they have ready access to a restroom. Everyday activities such as attending a theater or sporting event can become unpleasant. Sufferers often begin to avoid social situations in an effort to reduce the stress associated with their condition.

A variety of treatment options are currently available for treating urinary or fecal incontinence. Some of these include external devices, behavioral therapy (such as biofeedback, electrical stimulation, or Kegel exercises), prosthetic devices, artificial urinary and fecal sphincters (including the ACTICON® Neosphincter available from American Medical Systems), and surgery. Depending on the age, medical condition, and personal preference of a patient, surgical procedures can be used to completely restore continence.

One type of surgical procedure found to be an especially successful treatment option for urinary incontinence in both men and women, is a sling procedure. Sling procedures typically entail surgically implanting a biocompatible implant or "sling" to support the bladder neck. Sling procedures are discussed in U.S. Patent Nos. 5,112,344; 5,611,515; 5,842,478; 5;860,425; 5,899,909; 6,039,686; 6,042,534; 6,110,101; 6,478,727; 6,638,211; and PCT Publication Nos. WO 02/39890 and WO 02/069781. Some "pubomedial" sling procedures involve an abdominal incision and installation of a sling between the rectus fascia in the abdominal region to a position below the urethra, and back again to the rectus fascia. A conventional procedure in females is to surgically place a sling by entering the abdominal cavity through an incision in the patient's pubovaginal region.

In males, one example of a conventional method of treating urinary incontinence involves surgical placement of a sling by entering the abdominal cavity through an abdominal incision. Unfortunately, to access the abdominal cavity a surgeon must incise the male patient's abdominal muscles. This procedure is more time consuming and uncomfortable for the male patient.

Other methods for treating pelvic conditions involve installation of a sling below the urethra through incisions made at the inner thigh (e.g., in the perineal skin facing the obturator and in the groin), and using a tissue path extending through the obturator. These procedures can be referred to as "transobturator" methods. See, e.g., U.S. Patent No. 6,911,003 and U.S. Publication No. 2003/0171644A1.

WO 2005/122954 is directed towards an implant comprising soft tissue anchors and an adjustable length/tension sling. The sling is designed to treat urinary incontinence in both men and women caused by intrinsic sphincter deficiency and hypermobility.

WO 99/59477 is directed towards a tissue anchoring system. The tissue anchoring system includes a plunger assembly within a housing to advance an anchor into tissue.

WO 2006/108045 is directed towards an implant, tools, and methods to treat conditions such as prolapse and incontinence. The implant includes a tissue support portion, an elongate end portion, a dilator, and an elongate sheath.

While present methods of treating incontinence (e.g., fecal incontinence) can be effective, safe, and long-lasting, there is ongoing effort toward improving these methods.

### Summary

The present invention is defined by independent claim 1; the dependent claims describe embodiments of the invention.

Methods of treating fecal (a.k.a. anal) incontinence are not part of the present invention and may include surgical implantation of an implant (e.g., sling) to support tissue in a region of anal musculature, to improve fecal continence. As a single example of a useful placement of a fecal sling, a fecal sling can be placed at a position on an anterior side of the anus, which includes, e.g., tissue near the anal canal, external or internal anal sphincter, central tendon, puborectalis muscle (a part of the levator ani), corpus spongiosum, bulbospongiosus muscle, or at or near a combination of two or more of these tissues.

The implant includes a tissue support portion that supports tissue of a region of anal musculature, and an extension portion that connects to the tissue support portion and that attaches to a different location of the patient's anatomy to support the tissue support portion and, in turn, tissue of a region of anal musculature.

The extension portion can lead to an obturator foramen and be attached internally to the obturator foramen.

An end portion may be placed at or through an obturator foramen, an implant (sometimes referred to herein as "sling") includes two opposing elongate end portions that extend to and pass through each obturator foramen, and a tissue support portion (i.e., central support portion) that is placed to support tissue of a region of anal musculature. The tissue support portion can function to support or approximate tissue of a region of anal musculature, or nearby tissue. For example, tension can be placed on the implant to support or approximate one or more of the bulbospongiosus muscle, corpus spongiosum, external anal sphincter, internal anal sphincter, central tendon (i.e., perineal body), anal canal, and rectum. One possible result can be to recreate, support, or reposition, the anorectal angle of the rectum in a manner that improves fecal continence.

Exemplarily, the methods as described, for male or female anatomy may include a transobturator method that can involve, generally speaking, implanting a rectal (or "anal" or "fecal") sling with end portions of the sling passing through one or both of the opposing two obturator foramen, and with positioning and tensioning of a central support portion of the sling to approximate or support tissue of the pelvic region such as tissue of a region of anal musculature, and related pelvic tissue. Continence can be improved by approximating tissue of a region of anal musculature, optionally to re-align, approximate, or improve the alignment or positioning of rectal and anal tissue. Desirably, the sling can be tensioned to approximate and lift the rectal or anal tissue (e.g., posterior rectal or anal tissue) proximally, toward the bladder (in an anterior direction), and place or return the rectal or anal tissue to anatomically normal positioning, improving anal or rectal sphincter functioning, coaptation of the rectal or anal tissue, and continence.

According to certain exemplary methods for treating a male, a surgical installation can involve a medial incision at the perineum that exposes the bulbospongiosus muscle (also known as the BC or bulbocavernosum muscle), optional dissection of the bulbospongiosus muscle tissue to expose the corpus spongiosum, and placement of the central support portion in contact with the bulbospongiosus muscle or corpus spongiosum. Optionally the central support portion can be attached to the bulbospongiosus muscle or corpus spongiosum, e.g., by suture or other fastening mechanism. Tension can be applied to the end portions of the sling to approximate the bulbospongiosus muscle or the corpus spongiosum (CS), rectal or anal tissue (e.g., posterior rectal or anal tissue), or related tissues, in a proximal direction toward the bladder.

According to other exemplary methods in a male or female, a surgical installation can involve a medial incision at the perineum that exposes the bulbospongiosus muscle, optional dissection of one or more of the bulbospongiosus muscle and the central tendon, and placement of the central support portion in contact with the central tendon. Optionally the central support portion can be attached to the central tendon, e.g., by suture or other fastening mechanism. Tension can be applied to the end portions of the sling to approximate the central tendon and nearby tissues in a proximal direction toward the bladder, to improve continence.

Exemplarily, as a treatment for males and females, the methods can be used to treat stool incontinence or stool smearing (e.g., fecal incontinence) by perineal suspension, for example using a sling implanted by a transobturator method. A transobturator sling can be passed outside-in, or inside-out, in either a male or female, between incisions in a groin area ("lateral" incisions) to a medial incision. An "outside-in" approach dissects a tissue path by initiating a dissection (e.g., using a needle portion of an insertion tool) at the lateral incision and proceeding through the obturator foramen in the direction of the medial incision. An outside-in approach generally will include a next step of attaching an end portion of an implant to a needle distal end and retracting the needle back through the tissue path in a direction opposite the direction of dissection to pull the end portion of the implant back through the tissue path. An "inside-out" approach uses an opposite direction of dissection, initiating the dissection at the medial incision and proceeding through the obturator foramen in the direction toward the lateral incision. An inside-out approach may require alternate steps to install the end portion of the implant, such as attaching an implant at the handle end of the tool (after removal of the handle), and pulling the end portion of the implant through the tissue path in the direction from the medial incision to the lateral incision. Other alternatives are also useful, such as attaching the implant end portion to the leading edge of the surgical tool (at the needle distal end) before dissection, and pushing the end portion through the tissue path at the same movement of the dissection. As yet another alternative the tissue path can first be dissected, the needle can be removed (retracted), an end portion of an implant can be associated with the needle distal end, and the needle and end of an implant can be re-passed through the tissue path from the medial incision to the lateral incision.

A central portion of an implant (e.g., a tissue support portion) can be fixed to the perineal body (i.e., "central tendon") (or other tissue) and tensioned without or with fixating the sling ends to bone (e.g., inferior pubic ramii). The sling can preferably be passed high through the obturator fossae (e.g., through the upper third of the obturator foramen), in the process passing through the obturator externus muscle, obturator membrane, obturator internus muscle, and fascia. Tensioning the sling can be used to effect a proximal relocation of the central tendon area. This stretches the sphincter muscles around the urogenital sinus and anus, restoring fecal continence. This may lead to better sealing of the anal canal and tightening of anal sphincter muscles (both external and internal anal sphincter musculature). In a related embodiment the mesh can be anchored at the obturator and need not exit the body at an external incision.

Such methods may be useful, e.g., to treat stool smearing in elderly patients that may have occurred due to pelvic floor prolapse, in patients with spinal cord injuries, after spinal cord damage and surgery, in paraplegics, and in patients who suffer from congenital spinal cord disorders such as spina bifida leading to stool incontinence.

Exemplary minimally invasive procedures can be performed using a safe transobturator approach, using single or multiple incisions (as will be similar to a procedure for treating male urinary incontinence using an AdVance male urethral sling available from American Medical Systems (AMS) of Minnetonka MN), to effect proximal relocation (i.e., approximation in an anterior direction) of the perineal body (or "central tendon"). The method may result in stretching of the anal sphincter complex around the anus in a manner that restores continence of stool.

A sling can be installed using one or more tools to manipulate the sling to a desired position. Examples include curved two-dimensional or three-dimensional tools shaped to allow passage between a lateral incision (e.g., at the inner thigh or groin area) and a medial incision (at a perineum or vagina). End portions of the implant can be connected to or associated with ends of the needles, one for installing an end portion between a left-side lateral incision and the medial incision, through the obturator foramen. An opposing tool can assist to install the other end portion between the right-side lateral incision and the medial incision, through the obturator foramen. Examples of these types of tools are shown, e.g., at U.S. Patent No. 6,911,003 and at Published U.S. Patent Application Number 2003/0171644A1.
Optionally, a single tool may be used to install both sides of the sling through the left and right obturator foramen.

Exemplary left and right-handed tools can be designed with dimensions that are particularly suitable for installation of a sling using a transobturator method, in a male. These include relatively larger diameter and length of a three-dimensional portion, and a handle that provide an ergonomic advantage during a surgical installation procedure. These tools are described in U.S. Publication No. 2006-0235262-A1.

Also contemplated according to the present description are kits useful for commercial sale to surgeons, that include an implant and one or two installation tools adapted to install the implant using a method as described, such as a transobturator method. The sling and the tool or tools can be specifically designed to be useful for a male transobturator method, in that the tools can be designed with specific features of strength, and the sling can exhibit properties of increased strength, increased area of contact between the central support portion and tissue, and increased short-term and long-term fixation upon installation. See, e.g., U.S. Publication Nos. 2006-0287571 A1 and 2006-0235262 A1.

The invention is directed to a pelvic implant assembly as defined by claim 1, and systems and kits that contain the implant assembly.

The following patents and U.S. Patent Publications are additionally mentioned 7,070,556; 2002/0161382; 2002/0147382; 2004/0039453; 2005/0245787; 2005/0250977; 2006/0069301; 2006/0287571; 2006/0195007; 2006/0235262; 2006/0195010; 2006/0195011; and PCT Publication No. WO 2007/002012.

### Brief Description of the Drawings

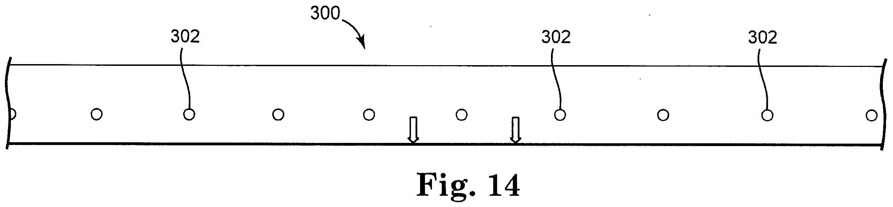

Figures 1 to 15 do not represent embodiments of the present invention.
Figure 1 illustrates general male pelvic anatomy and a location of an embodiment of rectal or anal sling according to the present description.
Figure 2 illustrates general male pelvic anatomy and a location of an embodiment of rectal or anal sling according to the present description.
Figure 3 illustrates general male pelvic anatomy and a location of an embodiment of rectal or anal sling according to the present description.
Figure 4 illustrates general female pelvic anatomy and a location of an embodiment of rectal or anal sling according to the present description.
Figure 5 illustrates general female pelvic anatomy and a location of an embodiment of rectal or anal sling according to the present description.
Figures 6A and 6B illustrate female pelvic anatomy, including locations rectal and anal tissues.
Figures 7A and 7B illustrate embodiments of tools useful according to the present description.
Figure 8 illustrates an embodiment of an implant useful as described herein.
Figure 9 illustrates features of an implant useful as described herein.
Figure 10 illustrates features of an implant useful as described herein.
Figure 11A illustrates exemplary material useful for preparing an implant for use as described herein.
Figures 11B and 11C illustrate features of an implant useful as described herein.
Figures 12A and 12B illustrate features of a tool useful as described herein.
Figures 13, 14, and 15 illustrate exemplary equipment and method steps useful to prepare an embodiment of implant useful as described herein.
Figures 16 and 17 illustrate exemplary self-fixating tips as described.

All figures are schematic and not necessarily to scale.

### Detailed Description

Surgical methods of the present description include methods of surgically implanting an implant such as a rectal or anal sling ("sling") to treat fecal or anal incontinence. The term "fecal incontinence" includes conditions sometimes referred to alternately or additionally as stool incontinence, rectal incontinence, anal incontinence, and the like, both in male and female patients.

A tissue support portion of an implant can be placed at a location to support tissue to improve fecal incontinence, such as at or near tissue of the anus, tissue generally anterior to the anus or anal canal (e.g., bulbospongiosus muscle, corpus spongiosum, puborectalis muscle, tissue of a perineal body, tissue of levator ani (i.e., anterior to anal tissue), or at tissue of an anorectal complex, including tissue of the rectum. The phrase "tissue at a region of anal musculature" refers to: tissue of a perineal body, a puborectalis muscle, a bulbospongiosus muscle, corpus spongiosum, an internal anal sphincter, an external anal sphincter, and combinations thereof.

Tissue of a region of anal musculature can be supported, as described, to improve fecal or rectal continence. The method of supporting the tissue can improve fecal continence by re-positioning, e.g., approximating, tissue of the pelvic region in a way that improves or increase the efficiency with which pelvic tissue independently or collectively functions to coapt the rectal or anal canal, without requiring a compressive effect on the rectal or anal canal itself. For example, the method may involve re-positioning tissue of the anorectal complex to improve continence. The "anorectal complex" includes tissue of a region of anal musculature (as described) and also includes tissue of the rectum, especially the lower portion of the rectum located proximal to the upper anal canal. Continence may be improved by any one or more of: changing the anorectal angle, and stretching or re-positioning tissue of the anal sphincter (external or internal), independently or in combination, in any manner that improves the ability of these and nearby tissues to function to coapt the anal canal.

The implant itself is supported, in turn, by attaching a portion of the implant such as an extension portion, to tissue of the pelvic region. As an example, an extension portion can extend from a tissue support portion located at a region of anal musculature, and be attached to an obturator foramen. Alternately, an extension portion can extend from a region of anal musculature and pass through an obturator foramen and extend to an external incision at an inner thigh or groin area.

Examples of the invention relate to surgical techniques, implants, tools, and related systems, kits, and assemblies, generally useful for treating fecal incontinence as described herein, including but not limited to methods that involve a transobturator technique. "Transobturator" methods generally involve two lateral incisions at the left and right inner thigh regions, each near a patient's obturator foramen, and a third, medial incision at the perineum in a male, or at a perineum or vagina in a female. The sling is installed between the medial incision and the two lateral incisions with a central support portion of the sling being placed at a location to improve fecal incontinence, such as at a region of anal musculature; the central support portion can be placed in a region below or anterior to the rectal or anal tissue, to support the rectal or anal tissue, not necessarily in contact with the rectal or anal tissue itself but optionally and preferably in contact with tissue below or anterior to the rectal or anal tissue. The sling can then be tensioned to reposition, approximate, or otherwise adjust the position or function of pelvic tissue to improve fecal continence.

Examples of methods, implants, and systems described herein can be used to treat incontinence in a manner that can differ from previous methods of treating fecal incontinence in males and females. Past methods such as installation of an artificial fecal sphincter (such as Acticon® from American Medical Systems), operate by a compressive effect on the sphincter that is meant to produce obstruction of the rectal or anal canal. In contrast, the invention can be used to treat fecal incontinence by re-positioning and approximating pelvic tissue in a way that improves or increase the efficiency with which pelvic tissues independently or collectively function to coapt the rectal or anal canal, without requiring a compressive effect on the rectal or anal canal itself.

A patient may suffer from pelvic tissue prolapse, weakness, or dislocation, due to one or more factors of age; weak, damaged, or sagging perineal floor muscles; or as a result of a surgical procedure such as a partial or radical prostatectomy, childbirth, hysterectomy, or for any other reason. Pelvic tissue prolapse may be in the form of mis-positioning of one or more component of pelvic tissue that makes up the rectal or anal sphincter complex.

Within a general concept of supporting (e.g., approximating or adjusting) pelvic tissue to treat fecal incontinence, a rectal or anal sling can be installed to approximate and support pelvic tissue, e.g., rectal or anal tissue, tissue of the perineal body, tissue of the rectal or anal sphincter complex (e.g., internal anal sphincter, external anal sphincter), etc., in any way that improves positioning or functioning of pelvic tissue to improve coaptation of the rectal or anal canal, resulting in improved continence. One skilled in the art can modify the description of the implant and the surgical methods provided herein to accommodate the implantation of a fecal sling as discussed, such as anterior to the anal or rectal canal or posterior to the rectal canal to improve positioning of pelvic tissue and organs, for both male and females, to improve fecal continence.

A central support portion of a sling may be placed anterior to the rectal canal and adjacent to bulbospongiosus muscle, and then tensioned to re-position pelvic tissue and improve continence. A rectal or anal sling can be installed in a male patient with the central support portion of the sling near the corpus spongiosum, optionally in contact with nearby tissue such as the rectal canal, to also improve or correct the ano-rectal angle to improve fecal continence. An anterior portion of a tissue support portion of a sling may be placed in contact with a posterior edge of a corpus spongiosum, with more posterior portions of the tissue support portion contacting tissue that includes a perineal body, and optionally tissue that is posterior to the perineal body.

In alternate methods, in a male or female anatomy, a central support portion of a sling may be placed in contact with one or more of the internal anal sphincter, the external anal sphincter, puborectalis muscle (which is part of the levator ani), or the central tendon (also referred to as the "perineal body").

In preparing a male or female pelvic region for placement of the central support portion at any of the described locations, the bulbospongiosus muscle may optionally be dissected, if desired, in a manner that will improve the mobility of tissue of the anorectal complex, better allowing movement in a manner that will improve fecal continence. Similarly, the central tendon may optionally be dissected, if desired, in a manner that will improve the mobility of tissue of the anorectal complex. Improved mobility may facilitate approximation of tissue of the anorectal complex in a manner to improve continence.

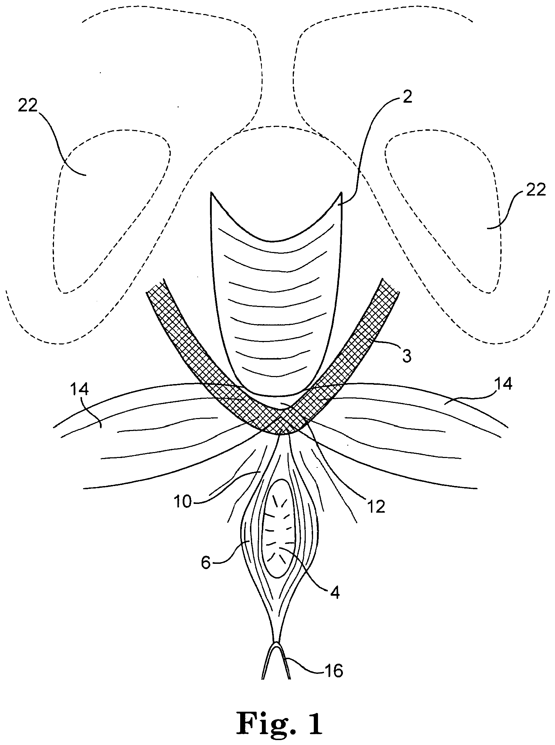

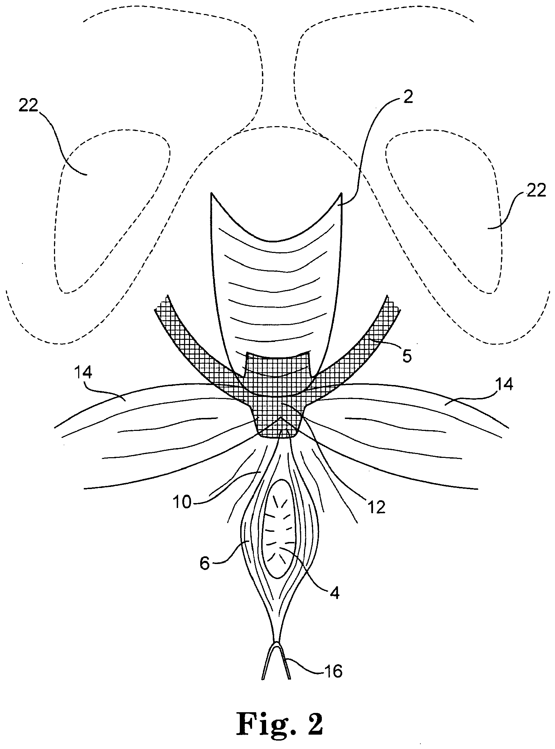

Certain relevant pelvic anatomy is illustrated at figures 1 and 2, which show tissue of the male pelvic cavity. Figure 1 shows tissues including bulbospongiosus muscle 2, anus 4, external anal sphincter 6, puborectalis muscle 10, perineal body 12, transverse perineal superficial muscles 14, coccyx bone 16, and left and right obturator foramen 22 (in shadow).

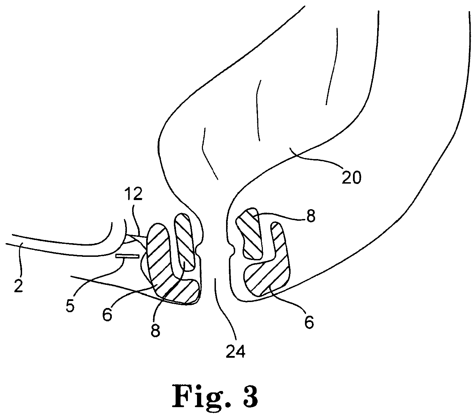

Figure 3 shows these anatomical features, in a male, in a side view, additionally illustrating internal anal sphincter 8, rectum 20 and anal canal 24.

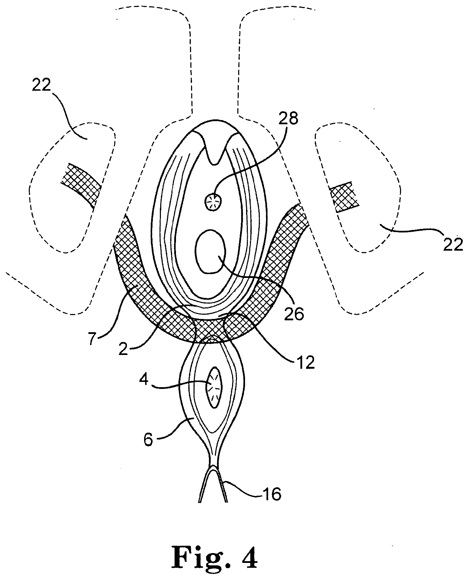

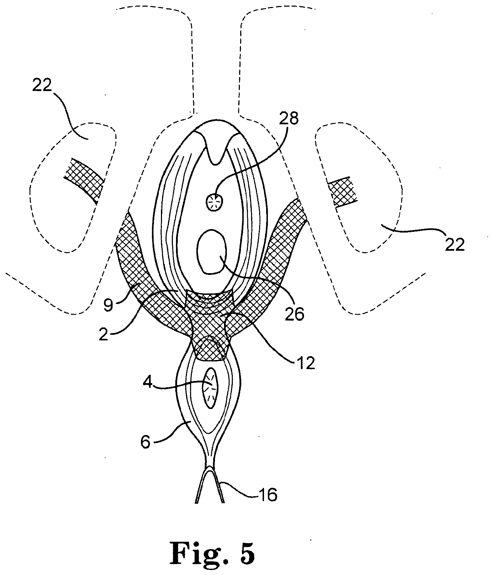

Figures 4 and 5 illustrate features in a female anatomy, additionally illustrating urethral meatus 28 and vaginal introitus 26, as well as implants 7 and 9, respectively.

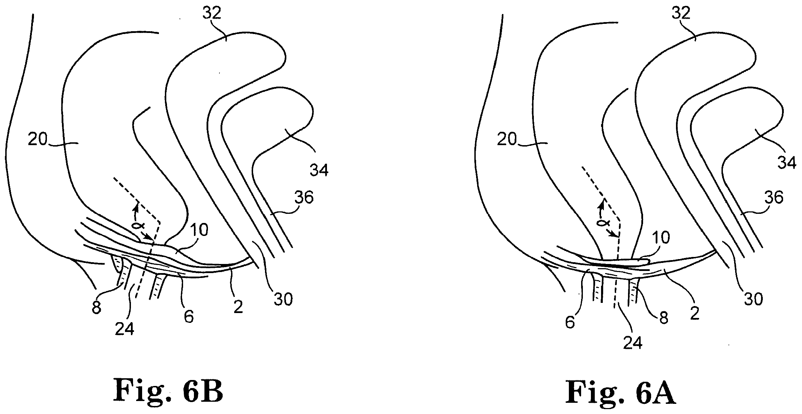

Figures 6A and 6B include these features in a female anatomy, additionally illustrating vagina 30, uterus 32, bladder 34, and urethra 36.

Referring to figure 1 fecal sling 3 can be placed in a position to approximate and support pelvic tissue to improve fecal continence. As illustrated, sling 3 is positioned in a male patient to contact perineal body 12 at a posterior position of perineal body 12, and optionally can contact tissue between the posterior portion of perineal body 12 and anus 4, such as one or more of: anterior portion of external anal sphincter 6, and tissue of puborectalis muscle 10. Optionally, sling 3 can additionally or alternately contact a posterior portion of bulbospongiosus muscle 2. (According to optional variations on this illustrated method, but not illustrated, bulbospongiosus muscle 2 may be dissected to expose corpus spongiosum, and implant 3 can be placed in contact with corpus posterior tissue of the spongiosum.)

Still referring to figure 1, according to exemplary methods, the implant can be tensioned by placing pressure on end portions of implant 3, in an anterior (and optionally upward) direction, to cause approximation of tissue of the anorectal complex in a manner to improve fecal continence. Approximation can include, e.g., movement of perineal body 12 in an anterior (proximal) direction.

Extension portions of implant 3 can be placed at any position to support the tissue support portion of the implant, which contacts tissue of the perineal body, puborectalis muscle, external anal sphincter, or bulbospongiosus muscle. Once positioned, an extension portion can be secured to tissue of the pelvic region to maintain the position of the central support portion and the approximated tissue of the pelvic region. The extension portion can be attached to tissue of the obturator foramen, can pass through tissue of the obturator foramen, or may be attached to tissue within or near a tissue path that extends from the perineal body to the obturator foramen. This tissue path may extend, for example, along a bottom surface of the levator ani tissue (below the tissue), between tissue of the levator ani and obturator internus muscle. The extension portion can be attached to tissue such as the anterior tissue of the levator ani, anterior tissue of the puborectalis muscle (which is part of the levator ani), tissue of an arcus tendineus, or tissue of an obturator internus muscle.

Referring to figure 2, illustrated is a modification of methods shown at figure 1. Figure 2 shows a method of treating incontinence using a fecal sling 5 that includes a widened central support portion. The widened central support portion can be placed in a position to approximate and support pelvic tissue to improve fecal continence. As illustrated, sling 5 is positioned to contact perineal body 12, a posterior position bulbospongiosus muscle 2, and tissue between the posterior portion of perineal body 12 and anus 4, including one or both of an anterior portion of external anal sphincter 6 and a portion of puborectalis muscle 10. As with the example of figure 1, the extension portions of implant 5 can be placed at any anatomical position that provides support for the tissue support portion of implant 5, to support tissue to provide improved fecal continence. (According to optional variations on this illustrated method, but not illustrated, bulbospongiosus muscle 2 may be dissected to expose corpus spongiosum, and implant 5 can be placed in contact with corpus spongiosum.)

Figures 4 and 5 show examples of methods as described, with regard to figures 1 and 2, respectively, modified to be performed on female anatomy.

According to exemplary methods as described, a method of surgically installing a fecal incontinence sling can include providing a medial incision at the perineum of a male or female patient to expose a bulbospongiosus muscle, optionally dissecting bulbospongiosus muscle (to expose corpus spongiosum, e.g., in a male), and placing a tissue support portion of a sling to contact tissue of a region of anal musculature. Optionally the tissue support portion of the sling can be fixed to tissue of the region of anal musculature, such as by use of a medical attachment in the form of a suture, staple, adhesive, clip, or the like. The implant can be tensioned to approximate tissue of the anorectal complex to improve fecal continence, and tension can optionally and preferably maintained chronically.

Figures 6A and 6B illustrate a method as described, particularly showing a useful effect of approximating tissue of an anorectal complex. Figure 6A shows a patient that exhibits sagging or weak pelvic musculature of a type that may be associated with fecal incontinence. Anorectal angle α refers to the angle, when viewed from a side of a patient such as in figures 6A and 6B, between the anal canal (which appears substantially vertical at figure 6A) and the rectum, which connects to the anal canal at an upper end of the anal canal and extends to a position that is above and posterior. (In the present description, the term "above," with reference to relative positions of human anatomy, refers to positions higher on the body of a standing human, i.e., toward the head, and the term "below" refers to positions lower on the body of a standing human, i.e., toward the feet.) In a correct anatomical position, an anorectal angle can be considered to be approximately 135 degrees, e.g., from 130 to 140 degrees. In a patient having reduced anal continence, the angle may be greater than the correct anatomical angle, such as from 140 degrees up to 180 degrees. This is shown in figure 6A.

Thus, examples of methods as described, for treating fecal incontinence, can include approximating tissue of the anorectal complex a manner that causes the anorectal complex to move to reduce the anorectal angle to an improved anatomical position, resulting in improved fecal continence. For example, if the anorectal angle of a patient suffering from symptoms of fecal incontinence is greater than about 140 degrees, exemplary methods of the invention can reduce the angle to an angle that is closer to 135 degrees. Figure 6B shows a patient after treatment according to this embodiment. At figure 6B, tissue of a region of anal musculature has been approximated and moved in an anterior direction, in an upward direction (toward the patient's head), or both anteriorly and upwardly. The tissue that is approximated to improve fecal continence may include one or more of a perineal body (not specifically identified at figures 6A and 6B), a puborectalis muscle (not specifically identified at figures 6A and 6B), bulbospongiosus muscle 2, corpus spongiosum, internal anal sphincter 8, and external anal sphincter 6.

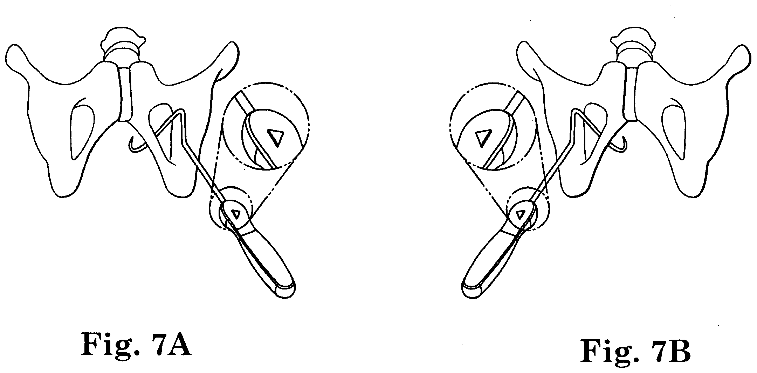

Figures 7A and 7B illustrate exemplary steps of installation of a fecal incontinence sling to treat incontinence according to a method of the present description that places ends of extension portions through opposing left and right obturator foramen, using external incisions at the inner thigh. The illustrated method can be referred to as an "outside-in" technique. Preferred locations for each tissue passage, through the left and right obturator foramen, are as high as possible on the obturator foramen, such as at the upper 1/3 of the medial part of the obturator foramen. A high passage may allow for application of maximum lift on the sling, or tissue of the region of anal musculature, etc., upon tensioning the sling.

Thus, a urethra sling can be used to correct for a loss of efficiency of the tissue of the anorectal complex, or misplacement of the anal canal and rectum, as can be associated with a non-natural anorectal angle. A fecal incontinence sling can be implanted, positioned, and tensioned to re-position and support pelvic tissue such as tissue of the rectum, anal musculature, bulbospongiosus muscle, corpus spongiosum, or other tissue that can affect fecal continence, to re-position misplaced pelvic tissue in a manner that improves fecal continence. The sling may be placed in contact with one or more of the corpus spongiosum, bulbospongiosus muscle, perineal body, external anal sphincter, internal anal sphincter, puborectalis muscle, etc., optionally after dissecting tissue of the bulbospongiosus muscle and optionally after dissecting tissue of the perineal body. The sling can be secured to the tissue that is contacted, and then tensioned to adjust and correct the position of tissue of the anorectal complex to improve functionality and coaptation of the anal sphincters and anal canal.

A sling for use according to methods described herein can generally be of the type useful as an implanted surgical device for treating male or female pelvic conditions, such as slings that are useful to treat urinary incontinence, either presently known developed in the future. Exemplary urethral slings that may be useful as described, or by modification, are discussed in U.S. Patent No. 6,911,003 and in U.S. Publication No. US 2003/0171644A1. Exemplary slings can be described as having two end portions (or "extension portions") extending in generally opposite directions from a central support portion.

A sling as described can be any implantable sling, known or developed in the future, that can be placed as described to treat fecal incontinence. The size and shape of the sling can vary depending on preference, installation considerations, anatomy, etc. Some embodiments of implants may include, for instance, a widened central support portion. Also, lengths of extension portions may be longer or shorter depending on the desired placement of the ends of extension portions, such as through an obturator foramen, at an obturator foramen, or elsewhere. Certain exemplary slings may be in the form of a uniform strip of mesh having a uniform width along its length, and being sufficiently long to reach from an external incision on a right inner thigh, through a right obturator foramen, to place a support portion in a region of anal musculature, then back through the left obturator foramen and to an external incision on a left inner thigh of a patient.

Dimensions of an implant can be as desired and useful for any particular installation procedure, treatment, patient anatomy, and to support or repair a specific tissue or type of tissue. Exemplary dimensions can be sufficient to allow the tissue support portion to contact tissue to be repaired or supported, and to allow extension portions to extend from the tissue support portion to a desired anatomical location to allow the extension portion be secured to anatomy of the pelvic region, to support the tissue support portion.

Dimensions of certain types of extension portions can allow an extension portion to reach between a tissue support portion placed to support pelvic tissue (at an end of the extension portion connected to the tissue support portion) and a location at which the distal end of the extension portion attaches to pelvic tissue. A distal end of an extension portion can optionally include a self-fixating tip that can be attached directly to pelvic tissue such as pelvic muscle, ligament, or tendon. The length of the extension portion, therefore, can be in a range that allows placement of a tissue support portion as desired to support pelvic tissue (e.g., at tissue of a region of anal musculature), while the self-fixating tip is placed in pelvic tissue.

According to certain examples, a length of an implant extending from one end to an opposite end (including the length of two extension portions and a tissue support portion) muss be sufficient to reach from an obturator foramen (preferably the upper 1/3 of the obturator foramen), to tissue of a region of anal musculature, and then to an opposite obturator foramen (preferably the upper 1/3 of the obturator foramen). This can be a total length for placing the sling in a male or a female anatomy. Each end includes a self-fixating tip for placement in tissue of the obturator foramen. In alternate examples the total length can be sufficient to extend between tissues at different locations at the anterior pubic region, such as from an arcus tendineus (at an anterior region of the arcus tendineus proximal to the obturator foramen), to tissue of a region of anal musculature, and then to the opposite arcus tendineus (at an anterior region of the arcus tendineus proximal to the obturator foramen); alternately from a puborectalis muscle (at an anterior region of the muscle proximal to the obturator foramen), to tissue of a region of anal musculature, and then to the opposite puborectalis muscle (at an anterior region of the muscle proximal to the obturator foramen). Exemplary such lengths can be, e.g., from 10 to 20 centimeters (not including self-fixating tips, if present), such as from 12 to 15 centimeters.

According to certain implants, a self-fixating tip can optionally include an aperture that allows passage of a guide, and can be used in combination with an insertion tool that includes an aperture that allows guided relative movement of the needle tip along the guide, as described in PCT/US07/022675. The guide may be a suture or other elongate plastic or metal guide that can be securely or loosely attached to the self-fixating tip. Methods of the invention can involve placement of a self-fixating tip at tissue of a pelvic region, with the guide contacting the self-fixating tip, such as by being threaded through an aperture of the self-fixating tip. The guide can be threaded through an aperture at an end of the insertion tool needle to allow the needle to be guided into contact with the self-fixating tip previously placed at tissue of the pelvic region. The position of the self-fixating tip can be adjusted, e.g., by further inserting the self-fixating tip. The guide may then be removed.

A preferred sling may include a widened central support, for increased contact and frictional engagement with tissue of a region of anal musculature. According to some examples, the sling can be tensioned to approximate corpus spongiosum proximally. A widened central support portion can provide improved mechanical and frictional engagement between the central support portion and tissue to be supported, e.g., bulbospongiosus muscle, corpus spongiosum, perineal body, etc., as described. A widened central support portion provides a larger area of contact between the sling and tissue in the pelvic region, and can have a reduced tendency to fold or deform upon tensioning of the sling. A suture can be used to attach the central support portion to tissue in the pelvic region to further improve the area of contact and prevent folding, such as at a location on the anterior side of the central support portion. A suture may also be useful to prevent movement of the sling relative to tissue to be supported, e.g., bulbospongiosus muscle, corpus spongiosum, perineal body, etc., during or after installation or tensioning. Exemplary pelvic implants that can include a widened central support portion are described, e.g., in U.S. Publication No. 2006-0195007.

A widened central support portion has a width that is greater than a width of the end portions, e.g., the width of the end portion at a location that is adjacent to a load-transfer portion. A central support portion that has a width that is greater than a width of the end portions can improve contact between the implant and tissue to be supported by the implant, e.g., tissue of a region of anal musculature. An increased width of a central support portion may take the form of one or two lateral extensions or "lobes" that extend laterally in at least one direction (an anterior direction) for contacting tissue being supported. An anterior extension supports tissue that is relatively anterior to a patient's anatomy compared to an otherwise similar central support portion that exhibits a smaller width. Alternately, a central support portion may include two lateral extensions in each of an anterior lateral direction and a posterior lateral direction, to contact tissue both anterior and posterior to a central support portion of a relatively more narrow width.

An increased width, e.g., in an anterior direction, can provide for increased contact and frictional engagement between a central support portion and pelvic tissue such as the central tendon, and tissue that lies anterior and posterior to the central tendon, such as the bulbospongiosus muscle, corpus spongiosum, puborectalis muscle, external anal sphincter, etc. A widened central support portion provides a larger area of contact between the sling and a tissue and can have a reduced tendency to fold or deform upon tensioning of the sling. Increased contact area between a central support portion and pelvic tissue can further allow for improved ability to re-locate or approximate tissue if desired during implantation of the sling and treatment and support of pelvic tissue by use of the sling.

Adjacent to a central support portion, and connecting the central support portion to one or preferably to both end portions, can be one or two load-transfer portions. The load-transfer portion exhibits a width that is greater than a width of an end portion, such as the width of the end portion at the location at which the end portion connects to the load-transfer portion. The load-transfer portion also includes a width that is less than the width of the central support portion. Functionally, the load-transfer portion allows a load placed across the central support portion, between the end portions, to be distributed across a width of the central support portion that is greater than widths of the end portions.

The central support portion is of sufficient length to support and optionally partially surround pelvic tissue, e.g., to support the tissue to treat fecal incontinence, such as to support tissue of a region of anal musculature. A width of a central support portion can be the same as or greater than a width of end portions and is sufficiently wide to provide contact area and frictional forces between a central support portion and a tissue in contact with the central support portion. Exemplary lengths of a central support portion can be in the range from 0.5 to 2 centimeters, such as from 1.2 to 1.8 centimeters. Exemplary widths of a central support portion can be in the range from 1.5 to 4 centimeters, such as from 2 to 4 centimeters. A combined length of two end portions, a central support portion, and one or more load-transfer portion or portions, can be approximately 16 inches (about 41 centimeters), e.g., within the range from 35 cm to 50 cm (these lengths may be suitable for methods that place extension portions through opposite obturator foramen and two external incisions in the inner thigh area). Alternate lengths can also be used, such as shorter lengths for methods that place end portions with self-fixating tips at internal locations.

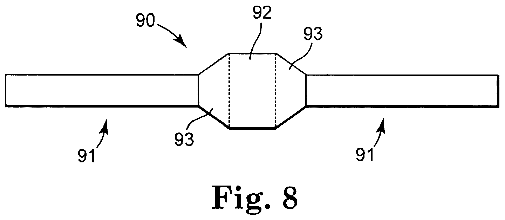

Figure 8 shows a sling 90 with a shape other than a purely rectangular shape. This example of sling 90 includes two end portions 91, a mid portion ("central support portion" or "tissue support portion") 92 that is wider than the remaining (e.g., end) portions of the sling 90, and load-transfer portions 93 connecting each of end portions 91 to central support portion 92. An anterior extension of central support portion 92 and load-transfer portions 93 extends laterally (in the width direction), in an anterior direction. A posterior extension of central support portion 92 and load-transfer portions 93 extends laterally and posteriorly.

A fecal incontinence sling may be integral, monolithic, or a composite of different components or segments of different synthetic or non-synthetic (e.g., "biologic") components. Suitable non-synthetic materials include allografts, homografts, heterografts, autologous tissues, cadaveric fascia, autodermal grafts, dermal collagen grafts, autofascial heterografts, whole skin grafts, porcine dermal collagen, lyophilized aortic homografts, preserved dural homografts, bovine pericardium and fascia lata. Suitable synthetic materials for a sling include polymerics, metals, and plastics and any combination of such materials.

Examples of synthetic sling materials include polypropylene, cellulose, polyvinyl, silicone, polytetrafluoroethylene, polygalactin, Silastic, carbon-fiber, polyethylene, nylon, polyester (e.g. dacron) PLLA and PGA. The sling material may be resorbable, absorbable, or non-absorbable. Optionally, some portions may be absorbable and other portions may be non-absorbable. Commercial examples of synthetic materials useful in a urethra sling include Marlex™ (polypropylene) available from Bard of Covington, RI, Prolene™ (polypropylene) and Mersilene (polyethylene terephthalate) Hernia Mesh available from Ethicon, of New Jersey, Gore-Tex™ (expanded polytetrafluoroethylene) available from W. L. Gore and associates, Phoenix, AZ, and the polypropylene sling available in the SPARC™ sling system, available from American Medical Systems, Inc. of Minnetonka, Minnesota. Commercial examples of absorbable materials include Dexon™ (polyglycolic acid) available from Davis and Geck of Danbury, CT, and Vicryl™ available from Ethicon. Other examples of suitable materials include those disclosed in U.S. Publication No. 2002/0072694,

A sling can include end portions that include side edges ("edges"), and the edges can optionally include edge extensions. Edge extensions refer to solid extensions of a non-uniform edge, interrupted by non-solid open spaces. Edge extensions can result from molding, cutting, or other formation of a solid or a porous our "open pore" end portion material. Exemplary edge extensions can be due to the open pore nature of a material used to prepare an end portion of a sling, and the process of cutting the material along a line that includes the pores to produce an edge.

For use according to the present description, edge extensions of an end portion of a sling can optionally be reinforced to cause the end portion to resist movement within tissue to increase short-term fixation properties of the sling. Reinforced edge extensions provide increased frictional resistance of an end portion from movement within the tissue, which provides desired short-term fixation properties of end portions within tissue during and immediately after installation, i.e., the ability of the end portions to stick and hold into flesh when installed without moving. Also preferably the end portions may be tensioned with without substantial stretching. According a first type of reinforcement, edge extensions can be reinforced by reinforcing open pore material adjacent to the edge (e.g., without treating the edge itself) in a way that limits movement of edge extensions and produces a stiffened edge extension. Other reinforcement can be in the form of a stiffening or reinforcing coating applied directly to edge extensions to limit the movement of the edge extensions. Reinforcement may also include combinations of treatments or structural features of edges or of areas of porous material adjacent to edges. Thus, a reinforcement may include or contact an edge (i.e., an end of an edge extension), may be adjacent to an edge but not include the edge (end of edge extension) itself, or may contact some portions along an edge of an end portion and not other portions along the same edge, while also including or contacting areas adjacent to the edge. With any of these reinforcements, the force required to pull a reinforced elongate strip through tissue can be increased. Exemplary implants having reinforced edge extensions are described, for example, in US patent publication nos. 2006-0287571-A1, 2006-0235262-A1, 2006-0195011-A1, and 2006-0195010-A1.

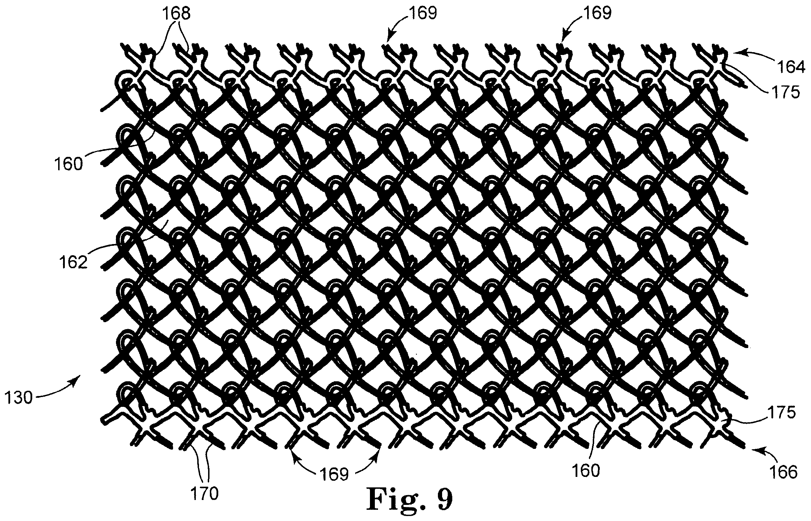

Exemplary reinforcement of an open pore mesh 130 is shown at figure 9. Figure 9 illustrates open pore mesh 130 with edge treatment of edge extensions (severed strands) 168 and 170. In figure 9, edge extensions 168 and 170 are reinforced by any one or more of a coating, heat treatment, or other mechanical or chemical treatment that stiffens edge extensions 168 and 170 at locations depicted by edge bands 175. Strand ends 169 are shown not to be reinforced.

One exemplary sling can be a strip or length of material of uniform width and thickness composed of interwoven fibers that can have spaced knots with or without heat set edges, such as the sling available under the MONARC™ trade name from American Medical Systems, Inc., of Minnetonka, MN. A sling may have stitching such as that of the MONARC™ sling, and may or may not include heat set edges or other reinforcement of edge extensions to increase strength of the sling or resistance to movement of the sling when the sling is in contact with tissue.

A central support portion is located between two opposing elongate end portions. The central support portion may be integral with the sling end portions as the same material, having the same width and thickness, or may be of another material having different width and thickness dimensions. The central support portion is attachable to or integral with the sling end portions and can be composed of a porous or non-porous plastic materials, a biologic material, or the like, such as a thermoplastic (e.g., polypropylene).

A synthetic sling or sling material may be prepared by any useful method, such as knitted, woven, sprayed, cut, punched from a blank, etc. Some slings may be sufficiently robust to be inserted without a protective sleeve. In other embodiments, some synthetic slings may have an associated protective sleeve to assist with the implantation. A useful mesh material may be prepared from one or more woven, knitted, or inter-linked filaments or fibers that form multiple fiber junctions throughout the mesh. The fiber junctions may be formed via weaving, knitting, braiding, bonding, ultrasonic welding or other junction forming techniques, including combinations thereof. The size of the resultant openings or pores of the mesh may be sufficient to allow tissue in-growth and fixation within surrounding tissue. As an example, not intended to be limiting, the holes may comprise polygonal shaped holes with diagonals of 0.132 inches and 0.076 inches.

The quantity and type of fiber junctions, fiber weave, pattern, and material type influence various sling properties or characteristics. As an example, not intended to be limiting, a useful mesh useful to construct a urethra sling may be woven polypropylene monofilament, knitted with a warp tricot. The stitch count may be 27.5 courses/inch (+ or - 2 courses ) and 13 wales/inch (+ or - 2 wales). The thickness of this example is 0.024 inches. This embodiment of sling is preferably associated with a protective sleeve. Non-mesh sling configurations are also included within the scope of the invention.

Exemplary sling materials or end portions of a urethra sling can be inelastic. A mesh or end portion of a urethra sling may be tested to determine whether it is elastic or inelastic using a series IX Automated Materials Testing System (an Instron), available from Instron Corporation. A 1cm wide sample of the mesh may be placed in the Instron with a crosshead speed set at 5 in/min and a gauge length of 1 inch. An elastic mesh exhibits at least a 7% elongation under a ½ pound load. An inelastic mesh exhibits less than a 7% elongation under a ½ pound load.

In one exemplary embodiment, a central support portion of a urethra sling can be substantially free of any silicone coatings. In yet another embodiment, a central support portion may comprise a non-synthetic material constructed according to the description of U.S. Provisional Patent Application No. 60/405,139, filed August 22, 2002. Other suitable synthetic slings are described in U.S. Patent No. 6,953,428.

A sling for use according to this description may have one or more substances associated therewith through a process such as coating or they may be incorporated into the raw material of the sling. Examples of appropriate substances include, without limitation, drugs, hormones, antibiotics, antimicrobial substances, dyes, silicone elastomers, polyurethanes, radiopaque filaments or substances, anti-bacterial substances, chemicals or agents, including any combinations thereof. The substances may be used to enhance treatment effects, reduce potential sling rejection by the body, reduce the chances of tissue erosion, enhance visualization, indicate proper sling orientation, and resist infection or other effects.

While a sling for use in fecal incontinence according to the present description may typically be elongate and rectangular, other variations are also contemplated. The size of a sling can take into account the imprecision associated with the range of human anatomy sizes. In one embodiment, a length of a sheath that covers an implant or a portion of an implant can be approximately within the range of 10 cm to 50 cm. A sheath width can be approximately within the range of 1.0 cm to 2 cm (e.g., from 1.1 cm to 1.5 cm wide), and sheath material thickness can be approximately within the range of 0.127 mm to 0.203 mm, respectively. An associated sling can have a length, width, and thickness approximately within the range of 7 cm to 50 cm; 1.0 cm to 2 cm; and 0.508 mm to 0.711 mm, respectively.

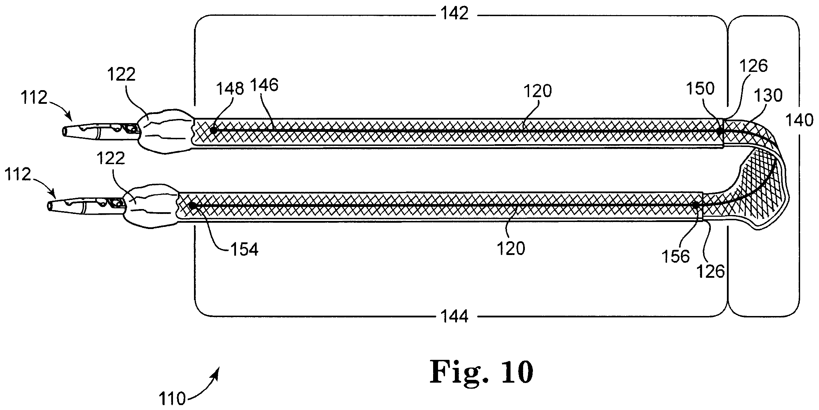

Referring to figure 10, an example of a sling assembly is depicted. Sling assembly 110 includes sling end connectors 112, which engage with free ends of right hand and left hand sling implantation tools (not shown). End connectors (e.g., "dilators") 112 can be shaped to dilate right and left passages through body tissue formed by curved needles of right and left hand implantation tools in a transobturator procedure. While not specifically illustrated, a sling as illustrated by figure 10 may include edge extension reinforcement as described above (e.g., a reinforcing coating, reinforcing weave, reinforcing strand, heat treatment, etc.).

Sling assembly 110 comprises sling 120 enclosed within protective sheaths 122 extending from sling end connectors 112 to free and open sheath ends 126. Preferably, protective sheaths 122 are constructed of a flexible thin transparent plastic film that enables visual examination of sling 120 and is sufficiently lubricious to pass easily through tissue passageways of a patient formed using sling implantation tools. Sheaths 122 can include sheath indicia or tear scores, perforations, or holes for assisting a surgeon in orienting sling 110 relative to tissue to be supported. Sling 120 can be left in place chronically following implantation, or optionally removed.

In alternate embodiments, a sling can include a connector that can be fixed directly to tissue of pelvic region, such as bone or soft tissue of muscle, ligament, tendon, etc. Exemplary connectors (e.g., self-fixating tips) are discussed, for example, at WO 2007/097994 A2.

According to the present invention, a "self-fixating tip" is attached at a distal end of an extension portion of an implant, and can be secured directly to tissue of the pelvic region, by placement within the tissue using a distal end of an insertion tool.

A self-fixating tip can be made out of any useful material, generally including materials that can be molded or formed to a desired structure and connected to or attached to an end of an extension portion of an implant. Useful materials can include plastics such as polyethylene, polypropylene, and other thermoplastic or thermoformable materials, as well as metals, ceramics, and other types of biocompatible and optionally bioabsorbable or bioresorbable materials. Exemplary bioabsorbable materials include, e.g., polyglycolic acid (PGA), polylactide (PLA), copolymers of PGA and PLA.

Exemplary self-fixating tips can include a cylindrical base or tapered cylindrical base, with a hollow or solid interior designed to engage a distal end of an insertion tool. Other shapes for a base may also be useful, such as blocks having square or rectangular forms when viewed in cross section along a longitudinal axis extending from a proximal base end to a distal base end.

As an example of a specific range of a length of a self-fixating tip, lengths (measured from the proximal base end to the distal base end along a longitudinal axis of the self-fixating tip) in the range from 0.4 to 1.0 centimeter, e.g., from 0.4 to 0.8 centimeters, or from 0.4 to 0.7 centimeters, have been found to be useful. These ranges are specifically useful for self-fixating tips that can be inserted into muscle of the pelvic region such as the obturator internus muscle, because the relatively short length can allow the self-fixating tip to be inserted into the muscle tissue a desired depth, i.e., over a range of depths, optionally without penetrating the obturator membrane; the self-fixating tip can be of a length dimension that is less than the thickness of the muscle or tissue into which the self-fixating tip is being inserted, so the self-fixating tip can be inserted a desired distance into the muscle or other tissue.

According to exemplary embodiments, a self-fixating tip can have structure that includes a base having a proximal base end and a distal base end. The proximal base end can be connected (directly or indirectly, such as by a connective suture) to a distal end of an extension portion of an implant. The base extends from the proximal base end to the distal base end and can optionally include an internal channel extending from the proximal base end at least partially along a length of the base toward the distal base end. The optional internal channel can be designed to interact with (i.e., engage) a distal end of an insertion tool to allow the insertion tool to be used to place the self-fixating tip at a location within pelvic tissue of the patient.

Alternate embodiments of self-fixating tips do not require and can exclude an internal channel for engaging an insertion tool. These alternate embodiments may be solid, with no internal channel, and may engage an insertion tool, if desired, by any alternate form of engagement, such as, for example, by use of an insertion tool that contacts the self-fixating tip at an external location such as by grasping the base (on a side or at the face of the proximal base end) or by contacting a lateral extension.

The self-fixating tip also, includes one or more lateral extensions that can increase the force required to remove the self-fixating tip from tissue after insertion into the tissue, i.e. the "pullout force." At the same time, the lateral extensions can be designed to exhibit a reduced or relatively low "insertion force," which is the amount of force used to insert the self-fixating tip into tissue. The self-fixating tip is designed to be essentially permanently placed upon insertion into tissue, or alternately can be designed to be used with a guide suture for initial placement and adjustment, as described in WO 2007/097994 A2 and PCT/US07/022675.

According to the present invention, the lateral extensions are "fixed" relative to the base so the lateral extension does not substantially move or deflect during or after implantation. For example, a fixed lateral extension can be a lateral extension that is not substantially moveable relative to the base in a manner that certain types of known soft tissue anchor extensions are moveable, for instance between a non-deployed or non-extended position that places an extension against the base to allow insertion of the anchor into tissue with a reduced size or shape profile, and a deployed or extended position that places the extension away from the base to engage tissue and prevent movement of the self-fixating tip in a direction opposite of the direction of insertion.

A lateral extension can have a three-dimensional form that results in a balance of the performance factors including insertion force, pullout force, and reduced trauma caused to tissue during insertion or in the event of a need to remove the self-fixating tip during an implantation procedure. A lateral extension can include a three-dimensional form referred to as an extension body defined as the lateral extension material between a leading edge, a trailing edge, and a boundary at which the lateral extension connects to a base; away from the boundary of the lateral extension and the base, the far lateral edge of a lateral extension may include a point of connection of the trailing edge and the leading edge, or another segment or connection may connect the leading edge with the trailing edge away from their respective connections to the base. The "leading edge" means the boundary of the lateral extension on the side of the lateral extension toward the base distal end, which is also the edge that leads the lateral extension body and contacts tissue first as the self-fixating tip is inserted into tissue by pushing. The "trailing edge" means the boundary of the lateral extension on the side of the lateral extension toward the base proximal end, which is also the edge that trails behind the lateral extension body and passes through or contacts tissue last when the self-fixating tip is inserted into tissue by pushing.

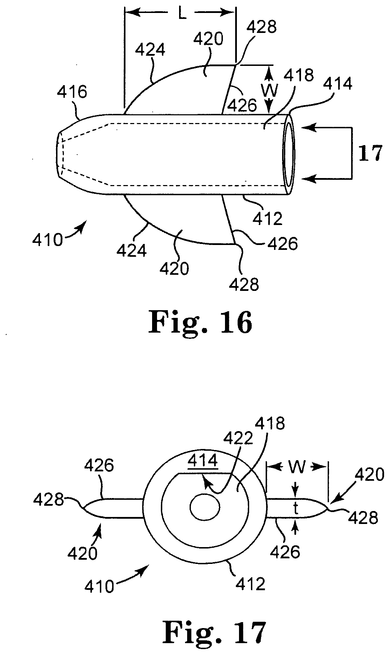

The lateral extension body can exhibit a thickness or thickness profile as desired, such as a uniform thickness or a varied thickness across the extended area of the body. For example, embodiments of implants may include a leading edge of a low profile, e.g., reduced thickness or even sharpened, to allow for reduced insertion force. According to these embodiments, the thickness of the lateral extension body can reduce gradually or taper from a central portion of the body (away from edges) in the direction of a leading edge. A leading edge, being of a reduced thickness to reduce insertion force, may optionally in addition exhibit a form that extends in a direction back toward the trailing edge, i.e., a "swept-back" leading edge, to further reduce insertion force. The shape of the leading edge may be linear or arcuate, and if arcuate may be convex or concave. Optionally the leading edge may take an arcuate convex path that sweeps back to meet the trailing edge at a single lateral extension point away from the base. E.g., see the exemplary self-fixating tip illustrated at figures 16 and 17.

The direction and shape of the trailing edge of a lateral extension, as the edge extends away from the base (e.g., when viewed as in figure 16), may be linear or arcuate, and if arcuate may be convex or concave relative to the lateral extension body. A trailing edge can be as desired, such as arcuate, straight, convex, flat, linear, rounded, tapered, sharp, blunt, etc. Optionally a trailing edge can exhibit a thickness (a thickness dimension is illustrated, e.g., at figure 17) to produce increased pullout force, yet that does not result in undue trauma in the event that a self-fixating tip must be removed from tissue after insertion.

Viewing the trailing edge along the longitudinal axis of the base and looking at the proximal base end (as in figure 17), a trailing edge can exhibit an area that includes a width (w, the distance the trailing edge extends laterally away from the base) and a thickness (t, the distance perpendicular to the width and the longitudinal axis of the self-fixating tip). An exemplary width (w, in figure 17) of the trailing edge can be, e.g., in the range from 0.5 to 3 millimeters.

An exemplary thickness at a trailing edge may be the same as a thickness at an interior or central portion of the lateral extension (away from the leading and trailing edges), or a thickness at a trailing edge may be a maximum thickness of the entire lateral extension, meaning for example that the thickness increases from a narrow thickness at the leading edge and widens gradually to a maximum thickness at the trailing edge. A thickness of a trailing edge can be, e.g., in the range from 0.2 to 2 millimeters, e.g., from 0.5 to 1.5 millimeters.

Based on the above-recited exemplary thickness and width dimensions, a surface area of a trailing edge may be, e.g., from the range from 0.25 to 5 square millimeters, e.g., from 0.5 to 4, or from 1 to 3 square millimeters. The surface area of the trailing edge may be concave, convex, rounded, tapered (symmetrically or toward one or the other surfaces of the lateral extension), etc. A flat surface may be preferred, to provide increased or maximum pullout force for preventing removal of the self-fixating tip after implantation.

A lateral extension can also include a third dimension that can be referred to as a "length" dimension (shown as "L" at figure 16). A length can be measured at a location where the lateral extension meets or extends from the base. This length dimension can become smaller as the lateral extension extends from the base. An exemplary length of a lateral extension at the location of the lateral extension meeting the base can be, e.g., from 0.5 to 5 millimeters, such as from 1 to 4 millimeters or from 1.5 to 3.5 millimeters.

In the specific example of a self-fixating tip for insertion to tissue of the obturator foramen, an exemplary length of a lateral extension can be a length that is less than the total thickness of obturator foramen tissue (i.e., the combined thickness of obturator internus muscle, obturator membrane, and obturator externus muscle); a length of a lateral extension intended to be inserted into the obturator internus muscle can be a length that is a portion of the thickness of the obturator internus, e.g., less than 1 centimeter, such as less than 0.5 centimeter.

A single example of a self-fixating tip, for purposes of non-limiting illustration and explanation, is at figures 16 and 17. Figure 16 shows self-fixating tip 410, including base 412 (for attachment to an implant extension end), proximal base end 414, distal base end 416, internal channel 418, and two lateral extensions 420 located on outer surfaces and on opposite sides of base 412. Tip 410 can be prepared from a biocompatible material, preferably a biocompatible material such as a biocompatible polymer, which may optionally be bioresorbable or bioabsorbable. Exemplary self-fixating tip 410 as illustrated includes internal channel 418 (optional according to the invention) which is an opening within base 412 extending from proximal end 414 toward distal end 416 along at least a portion of the total longitudinal length of base 412. Internal channel 418 is capable of receiving a distal end of an elongate needle of an insertion tool to allow tip 410 to be pushed into position within pelvic tissue during an implant installation procedure. Lateral extensions 420 include leading edge 424 and trailing edge 426. Leading edge 424 originates at base 412 and extends away from base 412 along an arcuate pathway sweeping back toward proximal base end 414, meeting trailing edge 426 at point 428. Leading edge 424 can preferably include a reduced thickness or a sharp or sharpened edge. Trailing edge 426 is shown to be relatively straight but could alternately be arcuate, concave, or convex. Trailing edge 426 has a flat surface area. Trailing edge 426 is also shown to sweep slightly back in a proximal direction, although it could alternately extend straight away from (i.e. perpendicular to) base 412 or extend away from base 412 in a direction that includes a forward component, i.e., a directional component in the direction of distal base end 416.

Referring now to figure 17, self-fixating tip 410 is viewed in a direction looking at proximal base end (surface) 414 along a longitudinal axis of base 412. In this view, surface areas of lateral extensions 420 are shown as flat surfaces of approximately the area of thickness (t) by width (w). Also visible in figure 17 is interior surface 422 of internal channel 418 of base 412. Surface 422 functions to orient self-fixating tip 10 in a desired rotational orientation relative to a distal end of a needle of an insertion tool; the distal end of the needle can be provided with a flat surface that is complementary to surface 422. As will be appreciated, surface 422 does not need to be a flat surface but could be any other type of surface or protrusion such as a rounded surface, angled surface, key structure, edge, or other feature that can orient a self-fixating tip rotationally relative to a distal end of an insertion needle.

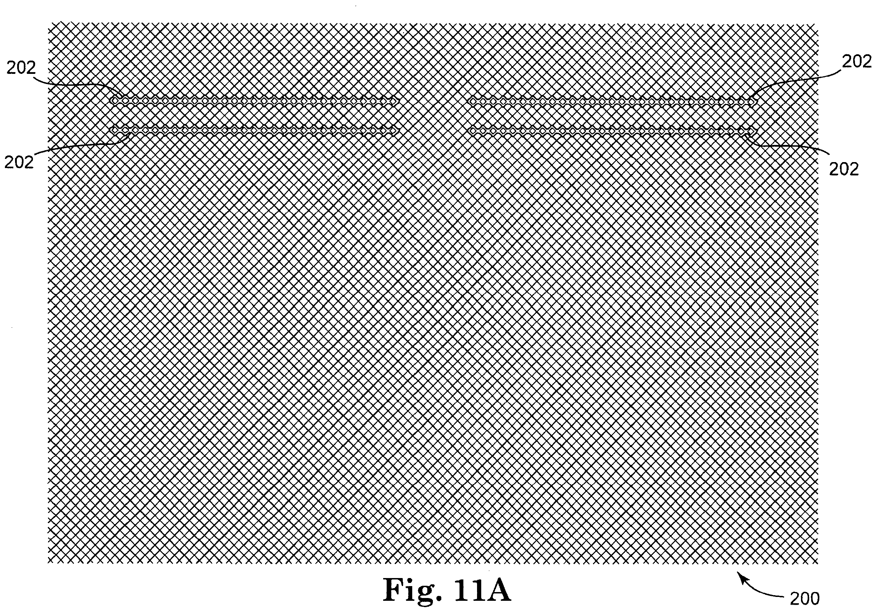

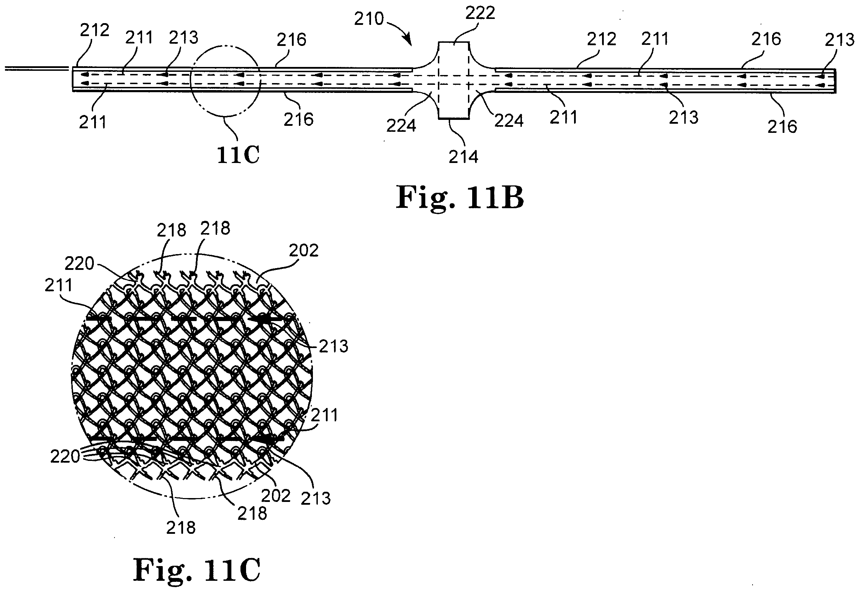

A specific example of a useful method for preparing an implant having reinforced edge extensions based on heat-treatment, is illustrated at figures 11A, 11B, and 11C. Figure 11A shows a sheet of open pore material 200, which is illustrated as a woven mesh but which may be any open pore material. Mesh sheet 200 is sized substantially larger than the total dimensions of a mesh implant that will be formed from sheet 200.

Figure 11A illustrates treated (e.g., heat-treated, coated, etc.) open pore material 202. Treated material areas 202 can be in the form of lengths of heat-treated open pore material (e.g., mesh) extending along a desired path of open pore material. As an example, heat-treated open pore material 202 may uniformly contact a longitudinal area that includes a series of adjacent pores along a length of mesh 200. Alternately or in addition, heat-treated material 202 may uniformly contact a longitudinal area that includes a series of adjacent junctions of mesh strands (e.g., knots) or other junctions or intersections of mesh 200. Contacting either a series of adjacent pores or junctions of a porous material can result in a uniform pattern of heat-treated material, e.g., a uniform length-wise area of heat-treated junctions, a uniform length-wise of heat-treated pores, or an area that includes pores and junctions.

In one specific example a heat-treated material 202 includes heat-treated junctions (e.g., knots or weaves) of a mesh material. With a location of heat treatment that includes a heat-treated junction of a mesh, cutting the mesh can be performed along a line that includes open pores that are immediately adjacent to and substantially parallel to the area that includes the series of heat-treated junctions. Upon such cutting step, edge extensions of non-heat-treated severed mesh strands can result at locations adjacent to elongate areas of heat-treated mesh junctions.

Figure 11B illustrates an example of a fecal incontinence sling cut from mesh 200 after formation of heat-treated material 202. Sling 210 includes two extension portions 212 extending from central support portion 214. Sling 210 includes a widened central support portion and two load-transfer portions, one on each side of the central support portion. The load-transfer portions are "bi-arcuate" load transfer portions, meaning that each of the two load transfer portions includes two arcuate edges: one extending in a posterior direction and one extending in an anterior direction. Sutures 211 (which are optional) extend the length of implant 210, attached at attachment points 213, which may be knots, adhesive, or another mode of attachment.

Extension portions 212 include edges 216 extending at the location of a cut made in mesh 200, following heat-treatment to form heat-treated material 202. Each of edges 216 includes edge extensions 218 and reinforcement in the form of heat-treated material 202. Figure 11C illustrates a close-up of edges 216, including mesh of extension portion 212, edge extensions 218 in the form of severed strand of un-heat-treated material, and heat-treated material 202 that includes a first row of fiber junctions (e.g., knots) 220 adjacent to edge extensions 218.

Referring to figure 11C, the distance of the reinforcement of edge extensions 218, i.e., heat-treated material 202, from edge 216, can be any distance that stiffens edge extensions 218, and may depend on factors such as the type of mesh, size of connecting strands of mesh, size of knots, and length of edge extensions. For purposes of illustration, the two length-wise strips 202 located along each edge 216 may be at least 0.05 centimeter (measured laterally, perpendicular to the length of the edge) from the severed ends of edge extensions 518, e.g., from 0.1 centimeter from the severed ends of edge extensions 518.

An implant can have dimension and shape features particularly useful when designed for a particular type of procedure, such as for a female transvaginal procedure, a female perineal procedure, a male perineal procedure, or any of these that places an end of an implant either: through an obturator foramen to an external incision (i.e., a transobturator procedure), at tissue of the obturator, or at a different pelvic location (e.g., puborectalis muscle, arcus tendineus, or adjacent tissue). An implant designed for any these different procedures can be designed with a different type of end portion, such as an end portion that has desired length, that includes or does not include a sheath or a reinforcing suture along a length of the end portion, or that includes a desired type of connector, dilator, or self-fixating tip at the distal end of the end portion.

A fecal sling can be installed as described herein with the assistance of surgical equipment, instruments, or tools that will be understood to be of assistance in performing the present surgical methods. Examples of surgical tools that may be useful include tools of the type described herein and in U.S. Patent No. 6,911,003 and published US patent application No. 2003/0171644A1, which generally include right and left-handed opposing helical installation tools.

Exemplary surgical tools can comprise a needle sized and shaped to extend between a medial incision or a location of a region of anal musculature, to a location at which an end portion of the implant is passed to, through, or anchored to. For example, a tool useful for placing an implant in a male, through a medial incision in a perineum, may be designed to either a) initially extend through an incision substantially adjacent a patient's obturator foramen and then through the obturator foramen to a medial incision, or b) initially extend through a medial incision and subsequently through the obturator foramen and then to an incision substantially adjacent a patient's obturator foramen. Preferably, a needle may comprise a pair of ends having surfaces for affording association with either an implantable sling material (e.g., the material itself, a dilator, a connector, a self-fixating tip, or a tissue anchor), or a removable handle. In one embodiment, a needle is sized and shaped for use on either the patient's right side or left side (not both). Optionally, a distal end of a needle may include an aperture for engaging a guide, such as a suture.

In other examples of methods, an implantation tool may be designed to reach from a medial incision to a location internal to the patient, to place an extension portion at a desired location, such as to anchor or secure the end portion, preferably without an external incision other than the medial incision. For example, a tool may be designed to reach from a medial (vaginal or perineal) incision, to place an end of an implant extension portion at tissue of the obturator foramen, e.g., by placing a self-fixating tip at tissue of the obturator internus muscle. Alternate locations of the end of the extension portion can be as described, such as at the arcus tendineus, levator ani, or puborectalis muscle (e.g., an anterior portion of puborectalis muscle, which is part of the levator ani).

Embodiments of installation tools can be straight, curved in two dimensions, curved in three dimensions, or can include combinations of straight and curved portions. Exemplary tools can include a substantially straight spacer portion emerging from an end of the handle portion preferably along a longitudinal axis of the handle. This helps afford convenient passage of the needle using an ergonomic wrist roll adopted by some surgeons.

A three-dimensional region of a needle can include a structure that can be described as a variable spiral or helix portion extending from the distal end of a straight spacer portion. A spiral portion can be variable as the angle of the spiral portion changes between the beginning of the spiral (e.g., the end of the spacer) and the distal end of the needle. The shape of the spiral portion can be designed to avoid over-insertion of the needle into the body, which helps avoid damage to the sensitive structures in this region of the body.

A useful needle can have dimension and shape features particularly useful in a desired type of procedure, such as for a female transvaginal procedure, a female perineal procedure, a male perineal procedure, or any of these that places an end of an implant through an obturator foramen to an external incision (i.e., a transobturator procedure), a procedure that places an end of an implant at tissue of the obturator, or a procedure that places an end of an implant at a different pelvic location (e.g., the arcus tendineus). An insertion tool designed for any these different procedures can be designed with a specific type of distal end, length, and curvature, to allow placement of an end portion of an implant at a desired location.

An insertion tool used to place an end of an implant through an obturator foramen can be of a length sufficient to extend from a lateral incision adjacent the anterior side of the pubic bone, through the obturator foramen portion of the pubic bone, to a position on the posterior side of the pubic bone, and to then emerge from a medial incision made between the patient's obturator foramen incisions. Alternate needles may be shaped to extend along the same tissue path in the opposite direction, entering at the medial incision and exiting at the lateral incision. A large number of different sizes, shapes, and dimensions of needles are suitable for the present invention.

An insertion tool used to place an end of an implant at tissue of an obturator foramen, e.g., by placing a distal end of an end portion of an implant at the obturator foramen with the use of a self-fixating tip, can be of a length sufficient to extend from a medial incision (vaginal or perineal), to the obturator foramen, such as along a bottom portion of levator ani muscle, and between levator ani muscle and obturator internus muscle, to a position on the posterior side of the pubic bone and at the obturator internus muscle.

In certain examples a tool includes a handle or a portion of a handle may exhibit a non-circular form when viewed along the longitudinal axis of the handle. The non-circular cross-section can be, e.g., an oval, rectangle, rhombus, etc., having one dimension "width" that is greater than the dimension perpendicular to that "width." The non-circular form will provide surfaces on the handle for a surgeon to place pressure onto and to achieve a grip. The non-circular cross-sectional form also defines a midplane that is a plane that includes the longitudinal axis of the handle and extends along the widest dimension of the handle when viewed in cross section.

A needle distal end of a tool (measured at the tip of the needle distal end) may be located at a position in space relative to the handle midplane and longitudinal axis, to provide the user with an ergonomic advantage. The ergonomic advantage may relate to useful or optimized (e.g., increased) amounts of force and control that can be applied at the needle distal end during the transobturator installation procedure, meaning amounts of force, sensitivity, and control that the user will have over the needle distal end when manipulating the handle using the midplane for leverage or grasping. As an example, a needle distal end may be located at an angle relative to the midplane to provide an ergonomic strength advantage or control advantage to a surgeon during particularly risky or sensitive portions of a surgical procedure, such as portions of a surgical procedure that involve using the needle distal end to dissect a tissue path through or near sensitive organs or tissues, e.g., traversing the obturator foramen. The angle between the needle distal end and the midplane may provide the surgeon with the use of maximum hand or wrist strength and maximum control and precision during manipulation of the needle distal end through a sensitive or risky tissue path, when applying pressure to a handle having a midplane. See U.S. Publication No. 2006/0235262.

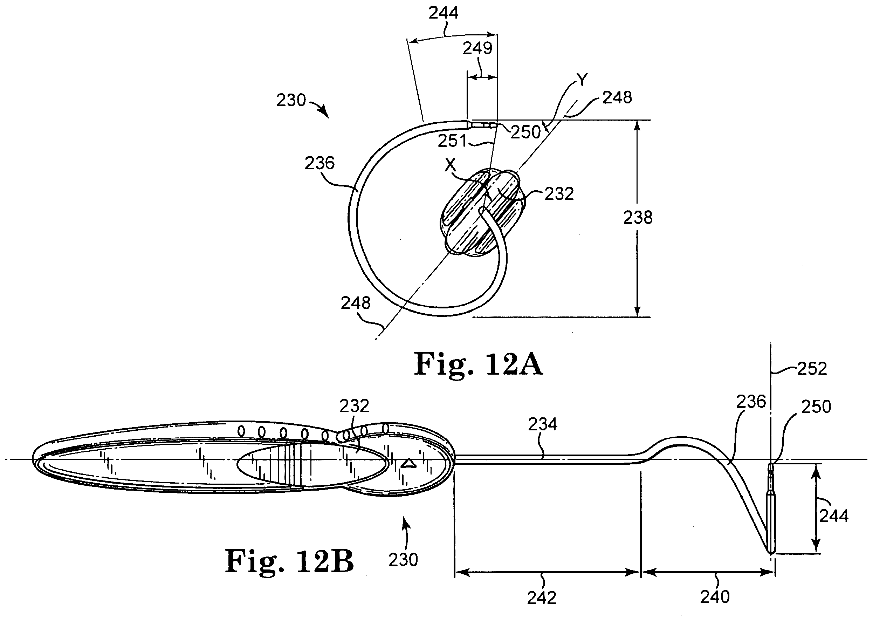

Figures 12A and 12B illustrate two views of a tool that involve a transobturator tissue path. Figure 12A illustrates a view of tool 230 along a longitudinal axis of the tool. Figure 12B illustrates a side view of tool 230. Tool 230 includes handle 232 and a needle extending longitudinally from an end of handle 232 along the longitudinal axis of the handle and tool. The needle includes spacer 234 and three-dimensional region 236 which may be considered to be a helix, a variable helix, or a spiral, etc. Diameter 238 can be larger than diameters of relevant prior art tools, and may be, for example, in the range from 2 to 5 centimeters, e.g., about 2.4 inches. Length 242 of spacer 234 can be any desired length, with an exemplary length 242 being in the range from 1 to 5 inches, e.g., from 1.75 to 2.25 inches. Length 240 of three-dimensional region 236 can be any desired length, such as in the range from 2.25 to 5 centimeters, e.g., from 2.4 to 2.5 inches. Angle y is approximately 45 degrees, and angle x is approximately 30 degrees, but may be otherwise, such as in the range from 20 to 70 degrees, or from 30 to 60 degrees. Needle end portion 244, which includes a length of about one inch at the end of the needle, is curved up until engaging portion 249, which is straight.

Figure 12B shows an axis of needle end portion, line 252, or a plane defined by the needle end portion, that is substantially orthogonal to the longitudinal axis of handle 232. Distal end portion 244 can define either a line or a plane, depending on, e.g., whether the distal end portion is straight or curved. In figure 12A, distal end portion 244 includes a curve, and as such defines a plane including needle distal end 250. This plane, illustrated as line 252, is substantially orthogonal to the longitudinal axis of tool 30. Radial distance 251 of tool 230 can be as desired, e.g., in the range from about from 0.7 to 1.4 inches, e.g., from 0.9 to 1.1 inch for a male transobturator tool. Also shown at figures 12A, needle end portion 244, which includes a length of about one inch at the end of the needle, is curved up until engaging portion 249, which is straight.

In yet another aspect, the present invention comprises a surgical assembly or kit for treating incontinence, the kit including any of the various implants as described, with any useful tool, for use according to a method, any of these being useful in combination with the others. An exemplary assembly can include an implant (sling) and one or two surgical instruments each having a handle portion, a needle portion having substantial structure in two or three dimensions, and a distal region. A kit can contain two surgical instruments (insertion tools) having structure in three dimension, e.g., a helical section. Each surgical instrument can include a handle portion and a needle portion having substantial structure in three dimensions and a distal region. The needle portion has a portion that is sized and shaped to extend between an incision substantially adjacent the obturator foramen on the patient's right side and a medial incision. The assembly also has a second surgical instrument for use on a left side of a patient. The second surgical instrument comprises a handle portion and a needle portion having substantial structure in three dimensions and a distal region. The needle portion of the second instrument has a portion that is sized and shaped to extend between an incision substantially adjacent the obturator foramen on the patient's left side and a medial incision.

Exemplary transobturator methods of installing a urethra sling include the steps of creating a medial incision at the external male perineum, creating two external opposing lateral incisions substantially adjacent the patient's left and right obturator foramen, and installing a urethra sling as described herein, end portions of which traverse the male obturator. The urethra sling may be placed using one or more tools as described, by installing end portions of the sling between the medial and the lateral incisions and passing through the obturator foramen. The end portion may be pushed through the tissue path at the leading edge of a needle, or may be pulled through the needle path using a trailing edge of the needle.

An exemplary method of treating fecal incontinence in a male or female, can include steps of creating a medial incision at the exterior perineum, creating an external lateral incision substantially adjacent the patient's obturator foramen, providing an elongate surgical instrument comprising first and second regions, the instrument having substantial structure in three dimensions, and providing an implant for treating fecal incontinence (a fecal incontinence sling). The three-dimensional region of the needle may be passed between the incisions, and then the implant can be associated with the instrument, e.g., at the end of the three-dimensional region. For example, the needle may be passed from the lateral incision through the obturator foramen and to the medial incision, and the implant can be associated with the tip of the needle extending from the medial incision. The needle can then be pulled back through the incisions to pull the end portion of the implant from the medial incision, through the obturator foramen, and to the lateral incision. The tissue support portion of the implant is placed a region of anal musculature in a manner to improve fecal continence.

Alternately, the implant can be associated with the needle before passing the needle between incisions. The needle, with the end portion of an implant associated with the needle tip, may then be passed between incisions, such as from the medial incision, through the obturator foramen, and then through the lateral incision. This can be done on both the right side and the left side.

In an alternate implantation method, a variation of a "transobturator" method includes inserting an implant through a medial, e.g., perineal incision and attaching an end portion of the implant to the obturator membrane (e.g., tissue of the obturator foramen, such as tissue of the obturator internus muscle) using a self-fixating tip, tissue anchor, soft tissue anchor, or other form of securing mechanism (e.g., a suture, biologic adhesive, staple, etc.). A self-fixating tip or a tissue anchor can traverse or otherwise attach to the tissue of the obturator foramen (e.g., obturator membrane, obturator internus muscle, etc.). These methods may use a tool that includes a needle curved in two dimensions (only) not necessarily three dimensions. Other features of the methods described herein can be incorporated into such a technique, such as placement of the tissue support portion of the implant as described herein at a region of anal musculature. This method avoids the need for lateral incisions. This method can be performed in male and female anatomies, using a vaginal or a perineal incision for a female anatomy. In yet other embodiments of the invention, an end portion of an implant (e.g., by use of a self-fixating tip or a tissue anchor, adhesive, staple, etc.) can be attached to any other tissue generally useful to support a tissue support portion of an implant at a desired location. Examples of useful tissue include tissues that are anterior to one or more of tissue of a region of anal musculature and tissue of an anorectal complex; these can include tissue of the arcus tendineus, levator ani muscle, puborectalis muscle, or nearby tissue.

In still another example of a transobturator method involving implantation using a needle with a three-dimensional region, a single needle may be useful to place left and right end portions both left and right sides of a patient. A single left-handed needle (alternately a single right-handed needle) can be used to place a right side of the sling on a patient's right side, using a transobturator tissue path between a perineal incision and a patient's right-side lateral incision. In the same procedure, the same left-handed needle may also be used to place the opposite end portion on the patient's left side. While the left-handed needle is not optimal for placement at the patient's left side, it can be effective. Systems or kits of the invention can include a single left- or right-handed needle with an implant, for surgical implant according to this method.

### Male Transobturator Sling System and Method

An exemplary sling system consists of two single-use surgical instruments called "needle passers" ("tool" or "needle") and a mesh implant with attached connectors, provided sterile. See figure 10. One end of each needle passer can be keyed to allow for secure placement of connectors or self-fixating tips at an end of the needle passer. Each needle passer has a plastic handle attached. The mesh is constructed of polypropylene monofilament that is precut to 1.2 centimeters arm width, 3.55 centimeters center width, and 35.5 centimeters length. Two absorbable tensioning sutures are threaded into the length of the sling system mesh to allow for tensioning adjustment of the sling system mesh after placement in the patient. Two plastic sheaths are placed over each arm of the sling system mesh to aid in ease of placement. The dilating connectors (alternately self-fixtating tips) are attached to the ends of the needle passers during the procedure. The mesh is intended to remain in the body as a permanent implant and the mesh component is not absorbed or degraded by the action of tissue in-growth or tissue enzymes.

The system is intended for the placement of a fecal incontinence sling system for the treatment of fecal incontinence (male or female). The following description is based on a male anatomy, but the methods described can also be useful in female patients.

The procedure can be carried out under local, regional or general anesthesia. A small vertical incision is made in the area of the perineum followed by periurethra dissection. Two small stab incisions are also made above the obturator foramen for needle entry.

### Preparation

1. Patient should be placed in a above lithotomy position.
2. Genital area should be shaved.
3. After shaving, the area should be scrubbed with Povidone-iodine soap for ten minutes or the approved hospital pre-operative scrub procedure.
4. Ensure that the bladder and rectum are empty.

### Dissection

1. The scrotum is elevated and a perineal incision is made, beginning midline at the level of the inferior edge of the symphysis and running approximately three centimeters toward the rectum.
2. The incision is carried deeper through Colles' fascia. The urethra is then mobilized by separating the bulbocavernosus muscle from the central tendon of the perineum.
3. The bulbocavernosus muscle is separated at the midline raphe and carefully dissected away from the corpus spongiosum.
4. A finger is placed between the bulbocavernosus muscle and the corpus spongiosum and with blunt dissection, the intersection of the corpus spongiosum and the perineal membrane is found.
5. The needle is inserted into the obturator foramen at a point bordering the inferior pubic ramus defining the foramen which lies approximately one-third of the distance below the forminal apex. Palpate the inferior pubic ramus and feel for the bony landmarks to locate the proper position. A needle through the skin can be used to probe the bone to help confirm that the correct location for the needle passer entry point is found, but it is not required. The position of entry is just below the medial aspect of the palpable part of the adductor longus tendon. The ideal position is at a point at the inner and medial aspect of the obturator foramen as high as possible to the foraminal apex.
6. Make small stab incisions at the correct location over both obturators (obturator foramina).
7. The patient is now ready for needle passage.

### Passing the Insertion Needle through the Obturator Foramen

1. Identify a three-dimensionally curved helical needle designated for the patient's left side.
2. Point the needle tip perpendicular to the skin and insert the needle into the patient's left stab incision previously made over the obturator foramen. The goal is to start with the needle tip hugging the medial aspect of the inferior pubic ramus within the obturator foramen at the level of the point one third below the cephalad peak of the obturator foramen.
3. Insert the needle to the level of the obturator fascia while hugging the bone with the needle tip.
4. Place an index finger in the perineal incision between the intersection of the corpus spongiosum and the perineal membrane on the side of the corpus spongiosum closest to the needle entry point.
5. When passing the needle on the patient's left side, keep the surgeon's right hand on the needle handle and left index finger in the perineal incision. The surgeon's left thumb should be on outside curve of needle to control the needle movement.
6. Using the left thumb on the outside curve of the needle for to control needle movement, push the needle through the muscles and obturator fascia by turning the needle handle clockwise using the right hand. The needle tip penetrates until resistance of the tissue stops -- about 0.5 centimeters.
7. Immediately locate the ischial pubic ramus with the needle tip and rotate the needle handle to allow the needle to follow the posterior ischial pubic ramus surface.
8. The index finger tip must palpate the needle tip while the needle is under the perineal membrane. The goal is to have the needle tip pass through the perineal membrane medial to the ischiocavernosus muscle, lateral to the corpus spongiosum and just below the level where the urethra passes through the perineal membrane. If not, move the needle to meet the finger tip. If the needle tip cannot be located, then the needle must be withdrawn just behind the ischial pubic ramus and carefully advanced again.
9. When the needle tip is in the correct position, guide the needle tip using the index finger through the perineal membrane until the needle extends through the incision.
10. Repeat the needle passage procedure (steps 2-9) on the patient's right side, with the needle designed for the right side.

### Placing the Sling System Mesh

1. Attach the connectors (that are pre-attached to the mesh) to the needle end. One connector should be attached to each of the needles on the end protruding from the perineal incision. Orient the knots of the tensioning sutures to be facing outward, away from the urethra. Be sure that the Sling System mesh lies flat and that the mesh is not twisted prior to attaching each connector.
2. Once both ends are connected, retract one needle along the same pathway, guiding with the fingertip.
3. Cut the insertion sheath and mesh at the external end of the plastic sheath and discard the needle, attached connector, sheath end, and mesh end. This step allows the sheath to slide freely relative to the mesh. Leave enough sheath material above the level of the skin so that the sheath can later be removed.
4. Repeat for the other needle on patient's contra lateral side to loosely position the sling with tensioning sutures facing outward, away from the urethra. Loosely position the sling with the center of the central portion of the mesh sling approximately 1 centimeter distal to the line created between the needle passages on both sides of the corpus spongiosum. Place the sling at the anterior of the rectal canal and anchor to that portion to facilitate repositioning of the rectal canal. In a related embodiment, the fecal sling is placed underneath the rectal canal to reposition the canal. This may also lead to reestablishing the ano-rectal angle but this is not necessary to reestablish continence in the patient. Other placement of the central support portion of the sling is also useful, as described, for improving fecal continence (e.g., placement at other locations to support tissue of a region of anal musculature).
5. In an optional step, before tensioning the sling, use two tack sutures to secure the placement of the sling to the midline of the corpus spongiosum. The sutures should be placed through the distal "flap" (anterior extension of the central support portion of the sling) just off of the center of the sling (at least two pores in from the edge of the sling mesh) and pass shallowly through the midline of the corpus spongiosum. When the sling is tensioned it will reposition the posterior urethra bulb approximately 1-4 centimeters proximal while elevating the perineal membrane.
6. The traction is parallel to the posterior urethra.

### Adjusting the Sling System Tension

1. If tissue retraction has been used, it must be removed before adjusting the tension of the Sling System.
2. The mesh is properly tensioned by simultaneously pulling on the ends of the sling mesh and noticing approximately 1-4 centimeters proximal movement of the urethra.

### To loosen the Sling System mesh:

Place an instrument between the sling mesh and the urethra. Ensure that both the mesh and the tensioning sutures are located beneath the clamp. Use the clamp to pull down and loosen the sling mesh as desired.

### To tighten the Sling System mesh:

Clamp a device such as a hemostat, across the sling mesh, at the lateral incisions. Be sure that both the tensioning sutures and the complete width of the sling are captured within the clamp. The sling mesh may be rolled around the clamp to improve the grip. Pull up to tighten the sling mesh as desired. If needed, this can be repeated on the contralateral side.

Remove the plastic sheath from the Sling System mesh and discard. Confirm the correct tension of the sling after the sheath has been removed.
Trim the sling mesh at the level of the subcutaneous tissue.
Complete a multi-layer closure of the perineal incision and the skin incisions.

### Immediate Post-Operative Care

◆ A catheter can be used at the discretion of the surgeon.
◆ Antibiotic prophylaxis should be given.
◆ The ability of the patient to empty the bladder should be confirmed.

### Example of Method of Preparation of Sling with Widened Central Support Portion and Reinforced Edge Extensions

Exemplary sling implants as described herein can be prepared according to the following, by the steps, in order, of (1) providing a sheet of mesh material, (2) heat treating the mesh to produce a heat treated area, and (3) cutting the heat treated mesh to form a urethra sling that includes reinforced edge extensions on end portions.

### Step 1 --- Heat Treating or "Sealing" Mesh

A sheet of polypropylene knitted mesh was provided for treatment in a heat-treatment or heat-sealing machine. The mesh was of the type used in the MONARC™ and SPARC® female urethra slings used for treating female urinary incontinence, from American Medical Systems, Inc., of Minnetonka MN. The mesh is that type that includes a "smooth" side and a "rough" side, as is known. The rough side may have a very slightly more rough feel compared to the smooth side; with reference to the direction of the loop that forms the weave, the loop points slightly more toward the "rough" side surface and slightly away from the "smooth" side surface. The "rough side" may be referred to as the "Technical Face" or "Loop Side" and the "smooth side" is called the "Technical Back" or "Lap Side". The invention can preferably apply heat ("sealing") at the Technical Back side of this type of mesh.

The pores are diamonds that have a size including an approximately 0.060" diameter measured (corner to corner) at the longer dimension and a 0.050" diameter measured in the shorter "width" direction (corner to corner). The sheet has rows of alternating diamonds that face up (the smallest angle point of the diamond faces up) adjacent to diamonds that face down (the smallest angle point of the diamond faces down).

The machine was turned on and set machine to the following cycle parameters:

| | |
|---|---|
| Temp of heated sealing element: | 395°F (±5°F) |
| Pressure applied to mesh by sealing element | 35 psi (±5 psi) |
| Time of pressure application sec) | 0.9 sec (±.1 |

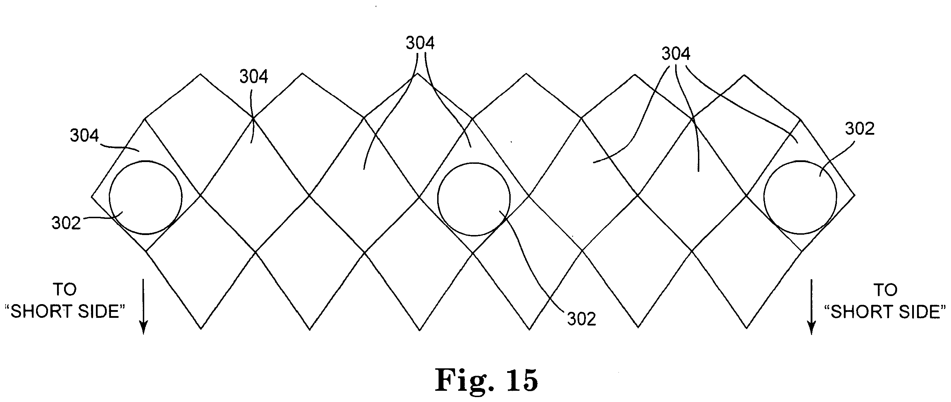

The mesh was loaded rough-side-down onto a plate insert that includes a line of several pins that are inserted into the pores of the mesh. The plate insert fits into a groove for positioning the plate and mesh below a heat treating element and a cutting die, for heat treating and cutting at locations of the mesh to produce heat treated reinforcement adjacent to edges, i.e., reinforced edge extensions. A portion of a plate is shown at figure 15, which shows plate 300 and pins 302 (not to scale). Pins 302 are not at the center of the width of the plate but are located closer to one side (referred to as the "short side," and indicated with the arrow) than the other side. This is because of the asymmetry of the "diamond"-shaped pores used to prepare the sling of the present example. The offset of the pins allows a cut of the mesh to align with pore openings as desired, and also allows heat sealing to align as desired, e.g., at a first junction of the mesh.

The mesh is aligned such that the pins of the plate are placed in the same row of pores of a mesh, with the pores being aligned along the length of the end portion as diamond-shapes as opposed to square-shapes (see figure 15). More specifically, because the diamonds of are asymmetrical, the diamonds are aligned with an orientation that points the smaller angle of the diamond in a direction away from the "short side" of the plate (indicated by arrows), i.e., the "diamond facing up" pores are held by pins 302. See figure 15, which schematically illustrates that pins 302 located to hold a single "row" of upward-facing diamonds 304, of with all diamonds held by pins 302 facing in the same direction.

A "mesh hold-down" piece is used to hold the mesh against the plate. The hold-down is made of Teflon and fits over the mesh and pins of the plate and does not otherwise interfere with the heating element contacting the mesh.

Load the mesh and plate into the heat seal machine, making sure the mesh is laying flat. Initiate heat treatment cycle with the parameters identified above.

### Remove Mesh Hold-Down.

### Step 2 --- Die Cutting the Sling

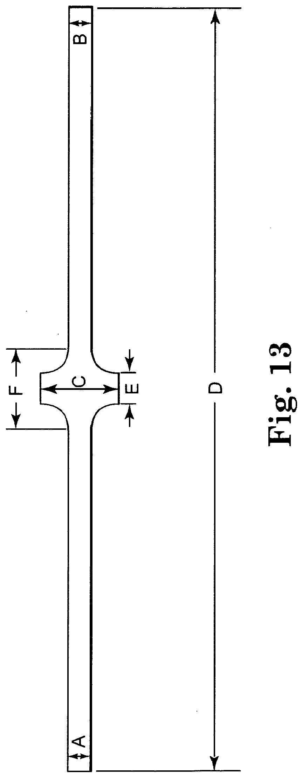

A pneumatic press, cutting die, plate insert and attached mesh (above) are provided. The die includes a blade that is shaped like a one-piece urethra sling, with the following dimensions, as shown in figure 13.

| **Dimension** | **Measured Value** |
|---|---|
| A | 0.44" |
| B | 0.44" |
| C | 1.4" |
| D | 14" |
| E | 0.58" |
| F | 1.5" |

The pneumatic press is set to 55 psi (±5 psi).

The plate with the mesh on it is placed into the cutting die. This lines up the cut to be adjacent to the heat-treaded portion of the mesh.

The die and mesh are placed in to the pneumatic press and the stamping cover with the plastic side down is placed on to the die.

The press is activated to cut out the sling.

If any strands of the sling did not cut, a pair of scissors can be used to separate the sling from the mesh panel along the cutting line of the die.

If necessary, edges of the sling may be cleaned with a bristled brush to remove any loose sling material.

## Claims

1. A pelvic implant assembly comprising
an implant (3, 5, 90, 110, 210) comprising a support portion (92, 140, 214) and two extension portions (91, 142, 212),
a self-fixating tip (410) connected at an end of each extension portion(91, 142, 212), each self-fixating tip (410) comprising
a base (412)comprising a proximal base end (414) and a distal base end (416), the proximal base end (414) being connected to the extension portion (91, 142, 212),
the base (412) comprising an internal channel (418) extending from the proximal base end (414) at least partially along a length of the base (412) toward the distal base end (416),
a fixed lateral extension (420) extending from the base (412),
wherein the total length of the support portion (92, 140, 214) and two extension portions (91, 142, 212) is sufficient for the implant (3, 5, 90, 110, 210) to reach from an obturator foramen, through a tissue path leading to tissue of a region of anal musculature, and through a tissue path leading to an opposite obturator foramen,
**characterized in that** the lateral extension (420) is fixed relative to the base (412) so the lateral extension (420) does not substantially deflect during implantation into pelvic tissue.

2. In combination, an implant assembly of claim 1, and an insertion tool (230) comprising a needle capable of engaging a self-fixating tip (410) and sized and shaped to extend through a medial incision to an obturator foramen.

3. The combination of claim 2 wherein the tool (230) comprises a needle curved in two dimensions.

4. A combination of claim 2 wherein the self-fixating tip (410) comprises an aperture capable of engaging a guide, a distal end of the needle comprises an aperture capable of engaging a guide, and the combination further comprises a guide.

5. An implant assembly according to claim 1 wherein the implant (3, 5, 90, 110, 210) is a fixed length.

6. An implant assembly according to claim 1 or 5 wherein the implant (3, 5, 90, 110, 210) is in the form of a uniform strip of mesh.

7. An implant assembly according to claim 1 or 5 wherein each self-fixating tip (410) is attached to a distal end of an extension portion (91, 142, 212).

8. An implant assembly according to any of claims 1 and 5 through 7 wherein the self-fixating tip (410) has a length measured from the proximal base end (414) to the distal base end (416), in the range from 0.4 to 1.0 centimeter.

9. An implant assembly according to claim 1 and 5 through 8 wherein the self-fixating tip (410) includes two or more lateral extensions (420), all lateral extensions (420) extending in a different direction from locations that are at the same length-wise position of the base (412).

10. An implant assembly of claim 9 wherein the self-fixating tip (410) includes exactly two lateral extensions.

11. An implant assembly according to claim 9 or 10 wherein the lateral extension (420) comprises a fixed leading edge (424) that extends away from the base (412) and proximally to meet a trailing edge (426) at a pointed lateral extension tip.

12. An implant assembly according to any of claims 9 through 11 wherein the lateral extension (420) comprises a lateral extension body comprising a leading edge (424) and a trailing edge (426), wherein the thickness of the lateral extension body tapers from a greater thickness at a central portion of the body to a reduced thickness at the leading edge (424).

13. An implant assembly according to any of claims 1 through 4 wherein the support portion (92, 140, 214) has a width and the two extension portions (91, 142, 212) have widths, and the width of the support portion (92, 140, 214) is greater than the widths of the extension portions (91, 142, 212).

14. An implant assembly according to claim any of claims 1 through 4 and 13 wherein
the implant (3, 5, 90, 110, 210) comprises a continuous mesh material having two mesh material ends, the support portion (92, 140, 214) and the two extension portions (91, 142, 212) being located between the mesh material ends,
each self-fixating tip (410) is connected fixedly to one of the two mesh material ends, and
the total length between the mesh material ends is in a range from 10 to 20 centimeters.

15. An implant assembly according to claim 14 wherein the total length between the mesh material ends is in a range from 12 to 15 centimeters.

## Patentansprüche

1. Beckenimplantatanordnung, welche aufweist:
ein Implantat (3, 5, 90, 110, 210), aufweisend einen Stützbereich (92, 140, 214) und zwei Verlängerungsbereiche (91, 142, 212),
eine selbstfixierende Spitze (410), die mit einem Ende jedes Verlängerungsbereichs (91, 142, 212) verbunden ist, wobei jede selbstfixierende Spitze (410) aufweist:
eine Basis (412), aufweisend ein proximales Basisende (414) und ein distales Basisende (416), wobei das proximale Basisende (414) mit dem Verlängerungsbereich (91, 142, 212) verbunden ist,
wobei die Basis (412) einen inneren Kanal (418) aufweist, der sich von dem proximalen Basisende (414) zumindest teilweise entlang einer Länge der Basis (412) zu dem distalen Basisende (416) hin erstreckt,
eine fixierte seitliche Verlängerung (420), die sich von der Basis (412) weg erstreckt,
wobei die Gesamtlänge des Stützbereichs (92, 140, 214) und der beiden Verlängerungsbereiche (91, 142, 212) ausreichend für das Implantat (3, 5, 90, 110, 210) ist, um von einem Opturatorforamen durch einen Gewebepfad, der zu Gewebe eines Bereichs von Analmuskulatur führt, und durch einen Gewebepfad, der zu einem entgegengesetzten Obturatorforamen führt, zu reichen,
**dadurch gekennzeichnet, dass** die seitliche Verlängerung (420) relativ zu der Basis (412) so fixiert ist, dass die seitliche Verlängerung (420) nicht wesentlich während der Implantierung in Beckengewebe ausgelenkt wird.

2. Kombination aus einer Implantatanordnung nach Anspruch 1 und einem Einsetzwerkzeug (230), aufweisend eine Nadel, die in der Lage ist, mit einer selbstfixierenden Spitze (410) in Eingriff zu treten, und bemessen und geformt ist, sich durch einen mittleren Einschnitt zu einem Obturatorforamen zu erstrecken.

3. Kombination nach Anspruch 2, bei der das Werkzeug (230) eine Nadel aufweist, die in zwei Dimensionen gekrümmt ist.

4. Kombination nach Anspruch 2, bei der die selbstfixierende Spitze (410) eine Öffnung aufweist, die in der Lage ist, mit einer Führung in Eingriff zu treten, wobei ein distales Ende der Nadel eine Öffnung aufweist, die in der Lage ist, mit einer Führung in Eingriff zu treten, und die Kombination weiterhin eine Führung aufweist.

5. Implantatanordnung nach Anspruch 1, bei der das Implantat (3, 5, 90, 110, 210) eine feste Länge hat.

6. Implantatanordnung nach Anspruch 1 oder 5, bei der das Implantat (3, 5, 90, 110, 210) in der Form eines gleichförmigen Maschenstreifens ist.

7. Implantatanordnung nach Anspruch 1 oder 5, bei der jede selbstfixierende Spitze (410) an einem distalen Ende eines Verlängerungsbereichs (91, 142, 212) angebracht ist.

8. Implantatanordnung nach einem der Ansprüche 1 und 5 bis 7, bei der die selbstfixierende Spitze (410) eine Länge, die gemessen von dem proximalen Basisende (414) zu dem distalen Basisende (416) in dem Bereich von 0,4 bis 1,0 cm liegt, hat.

9. Implantatanordnung nach einem der Ansprüche 1 und 5 bis 8, bei der die selbstfixierende Spitze (410) zwei oder mehr seitliche Verlängerungen (420) enthält, wobei sich alle seitlichen Verlängerungen (420) in einer unterschiedlichen Richtung von Orten, die an der selben Längsposition der Basis (412) sind, erstrecken.

10. Implantatanordnung nach Anspruch 9, bei der die selbstfixierende Spitze (410) genau zwei seitliche Verlängerungen enthält.

11. Implantatanordnung nach Anspruch 9 oder 10, bei der die seitliche Verlängerung (420) eine fixierte vordere Kante (424) aufweist, die sich von der Basis (412) weg und proximal erstreckt, um eine hintere Kante (426) an einer spitzen seitlichen Verlängerungsspitze zu treffen.

12. Implantatanordnung nach einem der Ansprüche 9 bis 11, bei der die seitliche Verlängerung (420) einen seitlichen Verlängerungskörper mit einer vorderen Kante (424) und einer hinteren Kante (426) aufweist, wobei die Dicke des seitlichen Verlängerungskörpers sich von einer größeren Dicke in einem mittleren Bereich des Körpers zu einer verringerten Dicke an der vorderen Kante (424) verjüngt.

13. Implantatanordnung nach einem der Ansprüche 1 bis 4, bei der der Stützbereich (92, 140, 214) eine Breite hat und die beiden Verlängerungsbereiche (91, 142, 212) Breiten haben, und die Breite des Stützbereichs (92, 140, 214) größer als die Breiten der Verlängerungsbereiche (91, 142, 212) ist.

14. Implantatanordnung nach einem der Ansprüche 1 bis 4 und 13, bei der
das Implantat (3, 5, 90, 110, 210) ein kontinuierliches Maschenmaterial mit zwei Maschenmaterialenden aufweist, wobei der Stützbereich (92, 140, 214) und die beiden Verlängerungsbereiche (91, 142, 212) sich zwischen den Maschenmaterialenden befinden,
jede selbstfixierende Spitze (410) fest mit einem der beiden Maschenmaterialenden verbunden ist, und
die Gesamtlänge zwischen den Maschenmaterialenden in einem Bereich von 10 bis 20 cm liegt.

15. Implantatanordnung nach Anspruch 14, bei der die Gesamtlänge zwischen den Maschenmaterialenden in einem Bereich von 12 bis 15 cm liegt.

## Revendications

1. Assemblage d'implant pelvien comprenant :
un implant (3, 5, 90, 110, 210) qui comprend une partie de support (92, 140, 214) et deux parties d'extension (91, 142, 212) ;
une pointe auto-fixante (410) qui est connectée au niveau d'une extrémité de chaque partie d'extension (91, 142, 212), chaque pointe auto-fixante (410) comprenant :
une base (412) qui comprend une extrémité de base proximale (414) et une extrémité de base distale (416), l'extrémité de base proximale (414) étant connectée à la partie d'extension (91, 142, 212) ;
la base (412) comprenant un canal interne (418) qui s'étend depuis l'extrémité de base proximale (414) au moins partiellement suivant une longueur de la base (412) en direction de l'extrémité de base distale (416) ;
une extension latérale fixe (420) qui s'étend depuis la base (412) ; dans lequel :
la longueur totale de la partie de support (92, 140, 214) et des deux parties d'extension (91, 142, 212) est suffisante pour que l'implant (3, 5, 90, 110, 210) atteigne un trou obturateur, au travers d'un trajet tissulaire qui aboutit à un tissu d'une région de la musculature anale, et au travers d'un trajet tissulaire qui aboutit à un trou obturateur opposé ;
**caractérisé en ce que** l'extension latérale (420) est fixe par rapport à la base (412) de sorte que l'extension latérale (420) n'est pas déviée de façon substantielle pendant une implantation à l'intérieur d'un tissu pelvien.

2. En combinaison, un assemblage d'implant selon la revendication 1 et un outil d'insertion (230) comprenant une aiguille disposant de la capacité d'engager une pointe auto-fixante (410) et dimensionnée et conformée de manière à ce qu'elle s'étende au travers d'une incision médiale jusqu'à un trou obturateur.

3. Combinaison selon la revendication 2, dans laquelle l'outil (230) comprend une aiguille incurvée selon deux dimensions.

4. Combinaison selon la revendication 2, dans laquelle la pointe auto-fixante (410) comprend une ouverture disposant de la capacité de recevoir un guide par engagement, une extrémité distale de l'aiguille comprend une ouverture disposant de la capacité de recevoir un guide par engagement, et la combinaison comprend en outre un guide.

5. Assemblage d'implant selon la revendication 1, dans lequel l'implant (3, 5, 90, 110, 210) est une longueur fixe.

6. Assemblage d'implant selon la revendication 1 ou 5, dans lequel l'implant (3, 5, 90, 110, 210) est sous la forme d'une bande de maille uniforme.

7. Assemblage d'implant selon la revendication 1 ou 5, dans lequel chaque bande auto-fixante (410) est liée à une extrémité distale d'une partie d'extension (91, 142, 212).

8. Assemblage d'implant selon l'une quelconque des revendications 1 et 5 à 7, dans lequel la bande auto-fixante (410) présente une longueur mesurée depuis l'extrémité de base proximale (414) jusqu'à l'extrémité de base distale (416) qui s'inscrit dans la plage qui va de 0,4 à 1,0 centimètre.

9. Assemblage d'implant selon l'une quelconque des revendications 1 et 5 à 8, dans lequel la pointe auto-fixante (410) inclut deux extensions latérales (420) ou plus, toutes les extensions latérales (420) s'étendant dans une direction différente depuis des emplacements qui sont à la même position de la base (412) en termes de longueur.

10. Assemblage d'implant selon la revendication 9, dans lequel la pointe auto-fixante (410) inclut exactement deux extensions latérales.

11. Assemblage d'implant selon la revendication 9 ou 10, dans lequel l'extension latérale (420) comprend un bord de tête fixe (424) qui s'étend en s'éloignant de la base (412) et de façon proximale de manière à ce qu'il rencontre un bord de queue (426) au niveau d'une pointe d'extension latérale effilée.

12. Assemblage d'implant selon l'une quelconque des revendications 9 à 11, dans lequel l'extension latérale (420) comprend un corps d'extension latérale qui comprend un bord de tête (424) et un bord de queue (426), dans lequel l'épaisseur du corps d'extension latérale va en s'amincissant depuis une épaisseur plus importante au niveau d'une partie centrale du corps jusqu'à une épaisseur réduite au niveau du bord de tête (424).

13. Assemblage d'implant selon l'une quelconque des revendications 1 à 4, dans lequel la partie de support (92, 140, 214) présente une largeur et les deux parties d'extension (91, 142, 212) présentent des largeurs, et la largeur de la partie de support (92, 140, 214) est plus importante que les largeurs des parties d'extension (91, 142, 212).

14. Assemblage d'implant selon l'une quelconque des revendications 1 à 4 et 13, dans lequel :
l'implant (3, 5, 90, 110, 210) comprend un matériau en maille continu qui comporte deux extrémités de matériau en maille, la partie de support (92, 140, 214) et les deux parties d'extension (91, 142, 212) étant situées entre les extrémités de matériau en maille ;
chaque pointe auto-fixante (410) est connectée de façon fixe à l'une des deux extrémités de matériau en maille ; et
la longueur totale entre les extrémités de matériau en maille s'inscrit dans une plage qui va de 10 à 20 centimètres.

15. Assemblage d'implant selon la revendication 14, dans lequel la longueur totale entre les extrémités de matériau en maille s'inscrit dans une plage qui va de 12 à 15 centimètres.
